# EUROPEAN PATENT APPLICATION

(11) **EP 2 987 802 A1**
(43) Date of publication of application: **24.02.2016**
(21) Application number: 14182006.8
(22) Date of filing: 22.08.2014
(51) Int. Cl.: C07K 14/435

(54) **An enzyme with chlorogenic acid esterase activity and feruloyl esterase activity**

(71) Applicant: Stern Enzym GmbH & Co. KG, 22926 Ahrensburg (DE)
(72) Inventor: NIETER, Annabel, 30167 Hannover (DE); LINKE, Diana, 31547 Bad Rehburg (DE); BERGER, Ralf Günter, 30455 Hannover (DE); THIESING, David, 20535 Hamburg (DE); POPPER, Lutz, 22527 Hamburg (DE)
(74) Representative: Moré, Solveig Helga

(57) **Abstract**

The present invention relates to a new enzyme having chlorogenic acid esterase activity and feruloyl esterase activity, i.e. having hydrolysis and synthesis activity of chlorogenic acid and hydroxycinnamic acid esters derived from the basidiomycete *Ustilago maydis,* UmChIE, and derivatives thereof. The invention also relates to nucleic acids, expression vectors, and host cells comprising the nucleic acid sequences as well as methods of preparing and using the enzyme, e.g., on synthetic as well as complex natural substrates, such as sugar beet pectin, destarched wheat bran and coffee pulp.

## Description

The present invention relates to a new enzyme having chlorogenic acid esterase activity and feruloyl esterase activity, i.e. having hydrolysis and synthesis activity of chlorogenic acid and hydroxycinnamic acid esters derived from the basidiomycete *Ustilago maydis,* UmChIE, and derivatives thereof. The invention also relates to nucleic acids, expression vectors, and host cells comprising the nucleic acid sequences as well as methods of preparing and using the enzyme, e.g., on synthetic as well as complex natural substrates, such as sugar beet pectin, destarched wheat bran and coffee pulp.

Plant cell walls consist of a complex set of polysaccharides including cellulose, hemicellulose and pectin. This polysaccharide network forms three-dimensional structures containing ester-linked hydroxycinnamates (1). These phenolic acids (caffeic, ferulic and p-coumaric acid) influence not only the rigidity and mechanical properties of the cell wall, but play also a role in plant defense (2). They are linked differently to sugar residues in plants: (A) in grasses such as wheat, barley or maize, ferulates and p-coumarates are mainly found esterified to the O-5 position of arabinose residues, whereas (B) in pectin of dicotyledons, such as spinach or sugar beet, ferulic acid is esterified to the O-2 position of arabinose and O-6 position of galactose residues (3). Furthermore, hydroxycinnamates also exist as soluble ester conjugates of quinic acid, with the best known example chlorogenic acid (4). Particularly high concentrations of chlorogenic acid are present in coffee, apple, pear and potato tuber (4). Especially health, cosmetic and pharmaceutical industries are interested in hydroxycinnamates due to their anticarcinogenic, anti-inflammatory and antioxidant properties (5). Consequently, enzymes able to release these phenolic compounds are of special interest for their potential industrial and medical application.

One group of these enzymes are feruloyl esterases (FAE; EC 3.1.1.73), a subclass of the carboxylic ester hydrolases (EC 3.1.1). They hydrolyze the ester linkage between ferulic acid or related cinnamic acids and complex plant cell wall polysaccharides (6). Over the years, a large number of FAEs were isolated and characterized mainly from bacteria, yeasts and fungi (7-10). Crepin et al. (10) developed a classification system for FAEs dividing them into four types (A - D) based on their substrate specificity supported by primary sequence identity. Type A FAEs are active on methyl ferulate (MFA), methyl p-coumarate (MpCA), methyl sinapate (MSA), but not on methyl caffeate (MCA). In contrast Type B FAEs hydrolyze MFA, M*p*CA and MCA, while Type C and D act on all four substrates. Only Type A and D are able to release diferulates from complex substrates.

However, there were only few studies focusing on chlorogenic acid hydrolase (EC 3.1.1.42). To date, a small number of chlorogenic acid esterases are published, all from *Aspergillus* sp.. Schöbel and Pollmann (11, 12) isolated and partially characterized a specific chlorogenic acid esterase from a pectinolytic enzyme preparation of *Aspergillus niger.* More recently, Asther et al. (13) demonstrated the efficiency of another *A. niger* enzyme to release caffeic acid from industrial by-products such as coffee pulp and apple marc. The corresponding gene was homologously overexpressed by Benoit et al. (14), and the properties of the recombinant and native enzyme were compared. Adachi et al. induced a chlorogenic acid esterase in mycelia of *Aspergillus sojae* with either instant coffee powder or coffee pulp (15). A hydroxycinnamic acid ester hydrolase from *Aspergillus japonicus* (16) and a cinnamate esterase from *A. niger* (17) also hydrolyzed chlorogenic acid. Additionally, activity towards chlorogenic acid was shown for the feruloyl esterase FAEB from *A. niger* (18), AoFaeB and AoFaeC from *Aspergillus oryzae* (19), TsFaeC from *Talaromyces stipitatus* (20) and Fae1A from *Anaeromyces mucronatus* (21). Couteau et al. (22) isolated and characterized human colonic bacteria acting on chlorogenic acid.

In light of this, the inventors solved the problem of providing a new enzyme having chlorogenic acid esterase activity and/or feruloyl esterase activity with alternative characteristics and substrate specificity which can be used, e.g., to generate the respective products. For example, hydroxycinnamate can be released from industrial by-products.

This problem is solved, e.g., by the subject matter of the claims.

The present invention provides specific uses of an enzyme having phenylpropanoid esterase activity, as well as specific phenylpropanoid esterase enyzmes. In particular, the invention provides specific uses of an enzyme with chlorogenic acid esterase activity and/or feruloyl esterase activity comprising a sequence having at least 85%, at least 90%, at least 95%, at least 96%, at least 97%, at least 98%, or at least 99% amino acid identity to SEQ ID NO: 1.

In a preferred embodiment, the invention provides an enzyme with chlorogenic acid esterase activity and/or feruloyl esterase activity comprising
a. a sequence having at least 85% amino acid identity to SEQ ID NO: 1, and not comprising SEQ ID NO: 3, or
b. a sequence having at least 85% amino acid identity to SEQ ID NO: 1 and not comprising SEQ ID NO: 19.

Throughout the invention, the enzyme preferably comprises a sequence having SEQ ID NO: 1, and is designated UmChIE. It may also consist of SEQ ID NO: 1. In some embodiments, the enzyme comprising SEQ ID NO: 1 does not comprise the endogenous signaling sequence of SEQ ID NO: 3. In other embodiments, it is the full length sequence of UmChIE provided by the inventors (SEQ ID NO: 17), including the signal sequence. In one embodiment, the present invention does not cover an enzyme comprising the sequence of SEQ ID NO: 19 (Um00182.1, GenBank accession no. **UP_756329.1)**. An enzyme with chlorogenic acid esterase activity and/or feruloyl esterase activity comprising a sequence having at least 85%, at least 90%, at least 95%, at least 96%, at least 97%, at least 98%, or at least 99% amino acid identity to SEQ ID NO: 1 may also comprise a leucine in position 492 of the sequence (calculated with reference to the positions in SEQ ID NO: 19).

Preferably, the enzyme has chlorogenic acid esterase activity, more preferably, the enzyme has chlorogenic acid esterase activity and feruloyl esterase activity.

Surprisingly, the inventors detected chlorogenic acid esterase activity in a basidiomycete, *Ustilago maydis,* and were able to isolate the responsible enzyme, UmChIE, despite problems with melanin production when standard nutrition medium was used. Only specific culture conditions, which induced higher esterase activities and avoided melanin production, allowed for purification of the enzyme. The enzyme was found to have an exceptionally broad substance specificity, as it has feruloyl esterase activity in addition to chlorogenic acid esterase activity. The enzyme was fully sequenced and characterized.

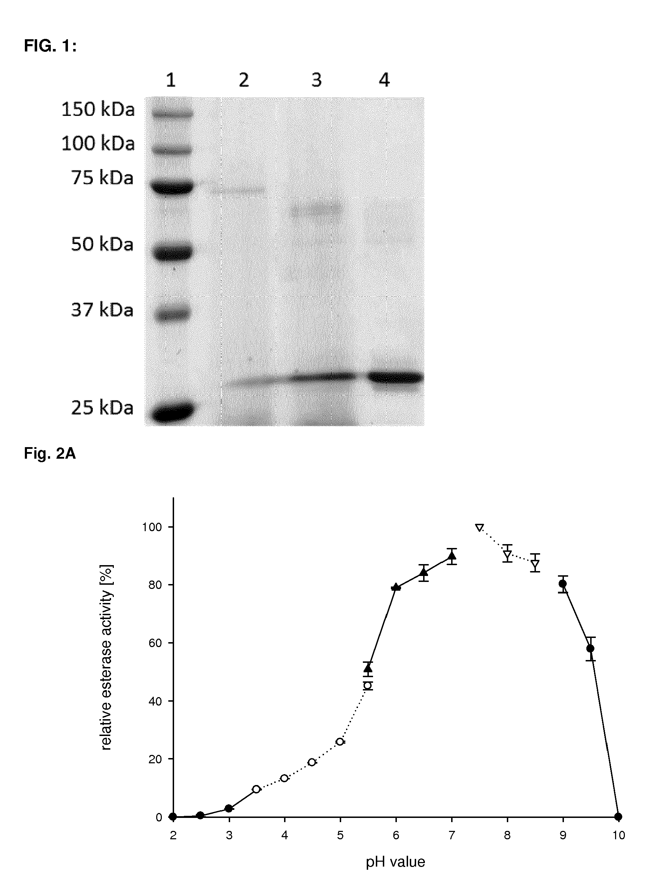

The molecular mass of the novel enzyme, as determined by denaturing SDS-PAGE, preferably is 71 kDA, as shown for the chlorogenic acid esterase isolated from *U. maydis* (Fig.1, lane 2). The apparent mass of the native enzyme estimated by size exclusion chromatography shows that UmChIE is a monomer. UmChIE thus differs from the other chlorogenic acid esterase enzymes described in literature. While Asther et al. (13) and Adachi et al. (15) identified the native chlorogenic acid hydrolases as homodimers of about 2 x 80 kDa, Benoit et al. (14) suggested the recombinant *A. niger* enzyme to exist as tetrameric form. Schöbel and Pollmann (12) characterized the second known chlorogenic acid esterase from *A. niger* also as a tetramer (4 x 60 kDa), whereas Okamura and Watanabe (16) described the hydroxycinnamic acid ester hydrolase of *A. japonicus* as a monomer of 145 kDa.

The sequence largely corresponds to the hypothetical protein Um00182.1 (SEQ ID NO: 19) from *U. maydis* 521 (GenBank accession no. **XP_756329.1)**, which was annotated via a genome project and roughly classified as a member of the esterase_lipase superfamily containing a region specific for carboxylesterases. No further information on that the sequence had been available before the present invention, e.g., there are no indications that the protein was ever prepared and isolated, and there was no information on its glycosylation or on its actual enzymatic activity.

The complete coding sequence of UmChIE as provided by the inventors has a length of 1,758 bp, corresponding to a protein of 585 amino acids (aa). The full aa sequence of UmChIE (SEQ ID NO: 17) purified from *U. maydis* by the inventors possesses three differences on nucleotide level which lead to one change on the amino acid level at position 492 (proline to leucine) compared to the sequence of Um00182. The UmChIE sequence showed highest similarity (89 %) and identity (81 %) to a carboxylesterase from *Pseudozyma hubeiensis* SY62 (GenBank accession no. **GAC96757).** In contrast, only 29 % identity was obtained with an aa sequence of the chlorogenic acid hydrolase CHIE from *A. niger* (GenBank accession no. **DQ993161).** A signal peptide of 21 aa was identified at the N-terminus of the UmChIE sequence based upon prediction using the web-based program SignalP 4.01. The predicted molecular mass of the mature protein was calculated to be 63.7 kDa using the Compute p*l*/Mw tool of ExPASy. The discrepancy to the molecular mass deduced from denaturing SDS-PAGE (71 kDa) indicated a glycosylated protein. Nine potential *N-*glycosylation sites were identified at positions 5, 109, 151, 298, 308, 370, 387, 487 and 558 (http://www.cbs.dtu.dk/services/NetNGlyc/). Treatment with endoglycosidase H results in a deglycosylated protein of approximately 63 kDa (Fig. 1, lane 3) suggesting that the UmChIE contains Asn-linked high-mannose oligosaccarides. Similar results were described for the chlorogenic acid hydrolase CHIE from *A. niger* (14). For CHIE a mass difference of 25 kDa was caused by glycosylation. Consequently, UmChIE has a lower degree of *N*-glycosylation than ChIE. This also explains the thermolability of UmChIE shown below, as it has been reported that *N-*glycosylation is important not only for protein folding, but also for thermostability (55).

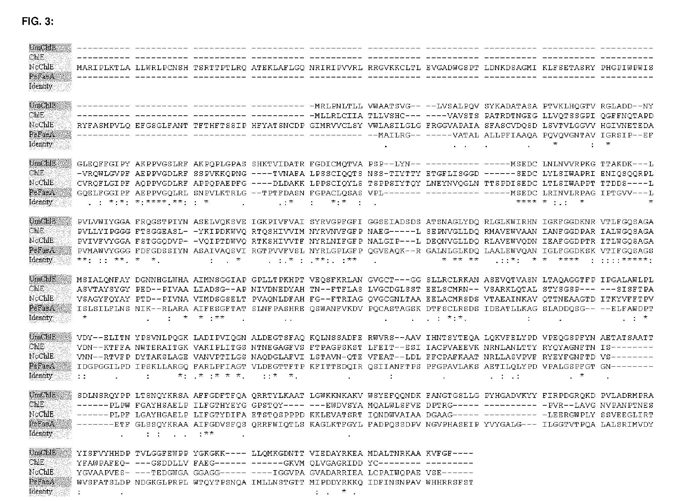

The sequence of the UmChIE contains a G-X-S-X-G motif (Fig. 3) characteristic of the serine esterase family (56, 57). Here, the motif G-Q-S-A-G is the same as in the CHIE sequence. This motif was also found in feruloyl esterases of *P*. *eryngii* PeFaeA (29) and *P*. *sapidus* Est1 (25), while other feruloyl esterase often possess G-H-S-L-G and G-C-S-T-G as conserved motifs (19, 58). The catalytic triad of UmChIE is predicted to consist of Ser228, Glu360 and His497 (http://pfam.sanger.ac.uk/). Likewise, CHIE contains a catalytic triad of Ser-Glu-His, while the annotated chlorogenic acid esterase from *N. crassa* (GenBank accession no. **EAA32507.3)** showed the glutamic acid replaced by aspartic acid (Fig. 3). Udatha et al. (48) reported a catalytic triad of Ser-Asp-His for 324 analyzed feruloyl esterase sequences. Nonetheless, two feruloyl esterases were also known containing a catalytic triad of Ser-Glu-His (25, 29). It could accordingly not have been predicted based on the sequence that UmChIE is a chlorogenic acid esterase.

The enzyme may have one or more of the following characteristics, preferably, all characteristics listed under a) to j), optionally, all of them:
a) non-gylcosylated or glycosylated at 1, 2, 3, 4, 5, 6, 7, 8, or 9 N-glycosylation sites;
b) monomeric;
c) chlorogenic acid esterase activity at about pH 3-10;
d) pH optimum of chlorogenic acid esterase activity at about pH 5.5-9.5, most preferably at about pH 7.5;
e) stability at pH 4-10;
f) feruloyl esterase activity on the substrates methyl p-coumarate, methyl ferulate, and methyl caffeate, but not methyl sinapate;
g) pl of about 3.0;
h) thermal stability up to about 40°C;
i) optimal activity at about 37°C;
j) optimal activity at about 0.1-3 M NaCl, preferably, 1-3 M NaCl or about 3 M NaCl; and/or
k) comprising a tag suitable for purification, such as a His tag, preferably, at the C-terminus.

In the context of the invention, the term "about" is intended to be understood as "+/- 10%). If "about" relates to a range, it refers to both lower and upper limit of the range. "A" is intended to mean "one or more", if not explicitly mentioned otherwise.

In some embodiments the enzyme is obtainable from a basidiomycete, preferably from the *Ustilago* genus, most preferably from *Ustilago maydis.* Homologues from other basidiomycetes are routinely obtainable for the skilled person, e.g., comprising using a probe from the nucleotide sequence encoding the enzyme of the invention for screening a cDNA bank of the basidiomycete under low stringency, medium stringency or high stringency conditions, thus isolating a homologous nucleic acid, and expressing the same in a suitable host cell. Alternatively, purification methods such as a combination of chromatographic steps, e.g., anion exchange chromatography on Q Sepharose FF, preparative IEF, and a combination of hydrophobic interaction chromatography and affinity chromatography, such as polyamide affinity chromatography can be employed to purify homologous enzymes from other basidiomycetes. Suitable methods are described in the examples in detail.

In other embodiments, the enzyme is obtainable by recombinant expression in a suitable host cell, e.g., in yeast, preferably in *Saccharomyces, Pichia, Kluyveromyces, Hansenula and Yarrowia* genera, more preferably in *Kluyveromyces lactis, Saccharomyces cerevisiae, or Schizosaccharomyces pombe,* and most preferably in *Pichia pastoris, e.g., P. pastoris* GS115.

The successful heterologous production of the active enzyme of the invention in *P. pastoris,* as described in detail below, opens up opportunities for applications and enzyme engineering. Identification of the catalytic centers and comparison with known enzymes further simplifies enzyme engineering, e.g., generation of mutants having only chlorogenic acid esterase activity or feruloyl esterase activity, having modified substrate specificity, or still better tolerance towards, e.g., low or high pH, salt, metal ions or high temperatures. In general, for engineered mutants which maintain the enzymatic activity of the UmChE, the catalytic centers and, preferably, residues which also contact the substrates, e.g., residues adjacent to the catalytic residues, or such residues which form the catalytic pocket, should be conserved.

The present invention also relates to a nucleic acid encoding the enzyme of the invention, i.e., an enzyme with chlorogenic acid esterase activity and/or feruloyl esterase activity having at least 85%, preferably, 90%, 95%, 96%, 97%, 98%, 99% or 100% amino acid identity to SEQ ID NO: 1. In particular, the invention relates to a nucleic acid encoding an enzyme with chlorogenic acid esterase activity and/or feruloyl esterase activity having at least 85%, preferably, 90%, 95%, 96%, 97%, 98%, 99% or 100% amino acid identity to SEQ ID NO: 1 not comprising SEQ ID NO: 4, or not encoding an enzyme comprising SEQ ID NO: 19.

The inventors surprisingly found that it was advantageous to use a heterologous signal sequence for expression. Accordingly, the nucleic acid of the invention preferably does not comprise SEQ ID NO: 4, the nucleic acid sequence encoding the native signal sequence. The nucleic acid of the invention may comprise the *U. maydis* sequence of SEQ ID NO: 2 encoding the enzyme of SEQ ID NO: 1 without the signal peptide, or consist thereof, or it may comprise the *U. maydis* full length nucleotide sequence SEQ ID NO: 18. Preferably, it does not comprise a sequence encoding the hypothetical gene bank protein Um00182.1 having SEQ ID NO: 19.

The nucleic acid of the invention may also encode a protein of SEQ ID NO: 1 with a sequence different from SEQ ID NO: 2 by virtue of the degeneracy of the genetic code.

The nucleic acid of the invention may be an expression vector comprising the nucleic acid encoding the enzyme of the invention, which is functionally linked to a heterologous promoter, e.g., an alcohol oxidase (AOX1) promoter, glyceraldehydes-3-phosphate dehydrogenase (GAP) promoter, alcohol dehydrogenase (ADH) promoter, or galactose (GAL) promoter, preferably, an alcohol oxidase promoter (AOX1). The nucleic acid encoding the enzyme is preferably functionally linked to a nucleic acid encoding the heterologous signal sequence, e.g., an α-factor secretion signal such as *Saccharomyces cerevisiae* α-factor secretion signal, an invertase secretion signal or a lysozyme secretion signal.

Functional linkage requires that the sequences are, insofar, as they encode amino acids, in frame, and in general, that they are positioned in a way that allows them to perform their respective intended functions. Accordingly, a promoter has to be positioned in a way leading to expression of the protein encoded by the functionally linked nucleic acid, and a signal sequence has to lead to secretion of the linked protein in the selected host cell. Optionally, the signal sequence can be cut off after secretion.

It the nucleic acid is not linked to a signal sequence, the expressed protein can be purified from the host cells, e.g., after lysing said cells. Alternatively, if the expressed protein is secreted into the culture supernatant, it can be purified from the supernatant.

A nucleotide sequence encoding for an affinity tag, such as a hexahistidine tag, may be incorporated at the C-terminus or N-terminus. This allows for easy purification of the enzyme of the invention, e.g., from the culture supernatant, with a suitable affinity column, e.g., an Ni-NTA. Alternatively, other purification methods, such as anion exchange chromatography on Q Sepharose FF, preparative IEF, and/or a combination of hydrophobic interaction chromatography and affinity chromatography, such as polyamide affinity chromatography, may be employed, or the cultures or culture supernatant may be employed as such.

The invention also relates to a suitable host cell, e.g., as mentioned above, comprising the expression vector of the invention. The host cell preferably is not *U. maydis,* i.e., the host cell is heterologous.

One surprising feature of the enzyme of the invention is its broad substrate specificity towards both chlorogenic acid esters and/or ferulic acid esters, which is described in detail in the examples below. Chlorogenic acid esterase activity and/or feruloyl esterase activity can be determined as described in the examples below. In particular, chlorogenic acid activity is determined by the capability of hydrolyzing chlorogenic acid. Feruloyl esterase activity is determined by the capability of hydrolyzing the 4-hydroxy-3-methoxycinnamoyl (feruloyl) group from an esterified sugar, which is usually arabinose.. An enzyme having the respective enzymatic activity may have at least 10%, at least 20%, at least 25%, at least 30%, at least 33,3%, at least 40%, at least 50%, at least 60%, at least 70%, at least 80%, at least 90% or at least 100% of the respective activity determined for the enzyme described in the examples below. It may also have higher activity.

The invention also provides the use of an enzyme with chlorogenic esterase activity and/or feruloyl esterase activity comprising a sequence having at least 85% to SEQ ID NO: 1 for hydrolyzing a substrate and/or generating a product selected from the group comprising:
(a) a chlorogenic acid, i..e, an ester of quinic acid with a phenylpropanoic acid selected from the group consisting of caffeic acid, ferulic acid and p-coumaric acid, preferably, caffeic acid; and/or
(b) an ester of a saccharide or C1- or C2-alcohol with a phenylpropanoic acid optionally comprising exactly one methoxy on the benzoic ring, wherein the phenylpropanoic acid preferably is caffeic acid, ferulic acid or p-coumaric acid, most preferably, caffeic acid.

Preferably, the enzyme is used for hydrolyzing one or more of said substrates, but the inventors found that under suitable conditions, the enzyme can also be used for the synthesis reaction.

The invention further provides a method of hydrolyzing a substrate selected from the group comprising:
(a) an ester of quinic acid with a phenylpropanoic acid selected from the group consisting of caffeic acid, ferulic acid and p-coumaric acid preferably, caffeic acid; and/or
(b) an ester of a saccharide or C1- or C2-alcohol with a phenylpropanoic acid optionally comprising exactly one methoxy on the benzoic ring, wherein the phenylpropanoic acid preferably is caffeic acid, ferulic acid or p-coumaric acid, most preferably, caffeic acid.

Said method comprises a step wherein an enzyme with chlorogenic esterase activity and/or feruloyl esterase activity comprising a sequence having at least 85% amino acid identity to SEQ ID NO: 1 is brought into contact with said substrate under suitable conditions, such as 37 °C, pH 7.5. The methods relating to the compounds under (a) are or special interest in the context of the invention.

The substrate may be a component of a natural material. If the substrate is a component of a natural material, the ester under (b) normally is an ester with a saccharide, such as a polysaccharide, e.g., di- or trisaccaride. The ester may, e.g., be a feruloylated mono-, di- and trisaccharides (F-A, F-AX, F-AXG). Further preferred substrates are described in the experimental part below.

The present invention further provides a method of preparing a product selected from the group comprising
a) quinic acid; and/or
b) a phenylpropanoic acid optionally comprising exactly one methoxy on the benzoic ring, preferably, caffeic acid, ferulic acid and/or p-coumaric acid, most preferably, caffeic acid; and/or
c) a saccharide such as a mono- or polysaccharide.

The products of group a) and b) and c) are products of the hydrolysis reactions catalyzed by the enzyme of the invention. Suitable conditions can be determined by the skilled person or are described in the examples below, e.g., 37°C, pH 7.5. As the enzyme can, under certain conditions, e.g., described in the examples below or the literature cited therein, also synthesize the esters, the present invention further provides a method of preparing a product selected from the group comprising
d) an ester of quinic acid with a phenylpropanoic acid selected from the group consisting of caffeic acid, ferulic acid and p-coumaric acid, preferably, caffeic acid; and/or
e) an ester of a saccharide or C1- or C2-alcohol with phenylpropanoic acid optionally comprising exactly one methoxy on the benzoic ring, e.g., caffeic acid, ferulic acid or p-coumaric acid, preferably, caffeic acid.

Said methods comprise a step wherein an enzyme with phenylpropanoic acid esterase activity, preferably, chlorogenic esterase activity and/or feruloyl esterase activity comprising a sequence having at least 85% amino acid identity to SEQ ID NO: 1 is brought into contact with the educts of the catalyzed reaction generating the respective product under suitable conditions.

Said methods optionally comprise a further step wherein the product is isolated. It may further be formulated, e.g., in a composition for cosmetic application or, preferably, in a pharmaceutical composition for medical application, e.g., to a human patient.

An extracellular halotolerant chlorogenic acid esterase from *U. maydis,* UmChIE, was purified to homogeneity and characterized, and the enzyme of the invention provided. Compared to previously described chlorogenic acid hydrolases said enzyme appears to display a broader substrate profile. Especially the ability to release not only caffeic acid, but also p-coumaric and ferulic acid from agro-industrial waste material, such as coffee pulp, renders this enzyme an attractive candidate for industrial applications. The phenolic acids liberated may be used as natural aroma precursors or antioxidants in food and cosmetics.

A recent study in mice did not confirm some of the supposed beneficial effects of dietary chlorogenic acid; quite the contrary of earlier positive indications was found (59). Depending on the outcome of this debate, the enzyme of the invention could provide a general and efficient tool for reducing dietary risks involved in the consumption of higher levels of chlorogenic acid.

The enzyme may be contacted with a natural material, e.g., food, which may be for human or animal consumption, such as apples, pears and potato tubers, or agro-industrial waste material, such as coffee pulp, destarched wheat bran, and sugar beet pectin. The invention thus provides a method of preparing natural material such as food comprising a reduced amount of an ester of quinic acid with a phenylpropanoic acid selected from the group consisting of caffeic acid, ferulic acid and p-coumaric acid, preferably, caffeic acid. The present invention also provides a method for reducing dietary risks involving the consumption of chlorogenic acid, in particular, high levels thereof, comprising contacting a food product with an enzyme with chlorogenic esterase activity and/or feruloyl esterase activity comprising a sequence having at least 85% amino acid identity to SEQ ID NO: 1.

The invention also relates to a food product, e.g., a baked food product or a food product intended for baking, comprising the enzyme of the invention.

Furthermore, the significant pH stability of UmChIE under alkaline conditions makes the enzyme particularly suitable for the treatment of pulp in the paper industry (paper pulp). It may be used alone or in combination with one or more different enzyme(s), e.g., a hemicellulase, a xylanase, a β-xylosidasen, an α-arabinofuranosidase or a pectin esterase. The inventors found that the simultaneous incubation of UmChIE with *T. viride* xylanase (0.025 U) increased the amount of released ferulic acid from DSWB more than twelvefold. This result can be explained by the action of endoxylanases producing shorter-chain xylo-oligosaccharides which are more accessible substrates for esterases.

In the methods and uses of the invention, all specific enzymes disclosed above may be used, including an enzyme having SEQ ID NO: 19.

All cited literature is herewith fully incorporated herein. The examples below are intended to illustrate the invention, but not to limit its scope.

### BRIEF DESCRIPTION OF THE DRAWINGS

FIG. 1 shows an SDS-PAGE analysis of the purified chlorogenic acid esterase (UmChIE) from U. *maydis* and endoglycosidase H treated enzyme. Precision plus ProteinTM Standard (1), purified UmChIE after polyamide column (2), deglycosylated UmChIE after treatment with endogylcosidase H for 1 h at 37 °C (3), endoglycosidase H (4).

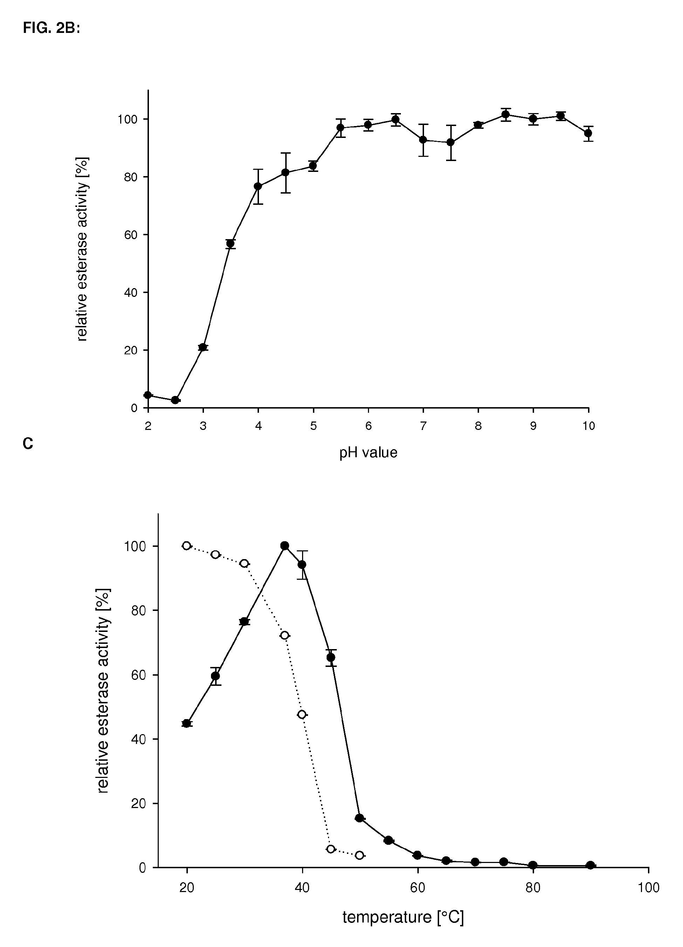

**FIG. 2** illustrates the effect of pH and temperature on the UmChIE activity. (A) pH optimum is determined using glycine buffer (filled circle), acetate buffer (open circle), Bis-Tris buffer (filled triangle) and Tris buffer (open inverted triangle); (B) pH stability; (C) temperature optimum (filled circle) and thermostability profile (open circle). For pH and thermostability measurements residual activities are determined after preincubating the enzyme for 1 h in the buffer systems above (pH 2-10) and at temperatures in the range of 20-60 °C, respectively. Remaining enzyme activity is determined after further incubation (1 h, 37 °C) with 1 mM methyl ferulate. Relative enzyme activity [%] is defined as the percentage of activity detected with respect to the maximum observed esterase activity in each experiment. Values are the average of triplicate experiments, with standard deviation shown as error bars.
**FIG. 3** illustrates the amino acid sequence alignment of *Ustilago maydis* UmChIE (GenBank accession no. HG970190) with chlorogenic acid esterase from *Aspergillus niger* ChIE (GenBank accession no. DQ993161), chlorogenic acid esterase from *Neurospora crassa* (NcChIE, GenBank accession no. EAA32507.3) and feruloyl esterase from *Pleurotus eryngii* PeFaeA (GenBank accession no. CDI44666.1). Peptide fragments identified by ESI-MS/MS- analysis are underlined. The catalytic triad of serine, glutamic/ aspartic acid and histidine is highlighted by boxes.

### EXAMPLES

### Materials and methods

### Chemicals and substrates

All chemicals used had highest purity grade and were purchased from Carl Roth (Karlsruhe, Germany), Merck (Darmstadt, Germany), Fluka (Buchs, Switzerland) or Sigma-Aldrich (Taufkirchen, Germany), unless otherwise noted. Methyl and ethyl ferulate were obtained from Alfa Aesar (Karlsruhe, Germany). Methyl caffeate, p-coumarate, and sinapate were synthesized according to Borneman et al. (23). Feruloylated saccharides (5-O-*trans*-feruloyl-L-arabinofuranose (F-A); *β*-D-xylopyranosyl-(1→2)-5-O-*trans*-feruloyl-L-arabinofuranose (F-AX); *α*-L-galactopyranosyl-(1→2)-*β*-D-xylopyranosyl-(1→2)-5-O-*trans*-feruloyl-L-arabinofuranose(F-AXG)) prepared from destarched corn bran were kindly supplied by M. Bunzel (KIT, Karlsruhe, Germany). These esterase substrates were purified and identified as described (24, 25). Sugar beet pectin was from Herbstreith & Fox (Neuenbürg, Germany), coffee was from Tchibo (Hamburg, Germany) and wheat bran was from Alnatura (Lorsch, Germany). PCR primers were synthesized by Eurofins MWG Operon (Ebersberg, Germany).

### Cultivation of Ustilago maydis (Uma)

The basidiomycete *U. maydis* was purchased from the Deutsche Sammlung fur Mikroorganismen und Zellkulturen (DSMZ, Braunschweig, Germany; No. 17144). The strain was grown on standard nutrition liquid (SNL)-agar and maintained at 4 °C. SNL medium was 30.0 g L⁻¹ D-(+)-glucose-monohydrate, 4.5 g L⁻¹ L-asparagine monohydrate, 3.0 g L⁻¹ yeast extract, 1.5 g L⁻¹ KH₂PO₄, 0.5 g L^{- 1} MgSO₄; 1.0 mL L⁻¹ trace element solution (0.08 g L⁻¹ FeCl₃ x 6 H₂O, 0.09 g L⁻¹ ZnSO₄ x 7 H₂O, 0.03 g L⁻¹ MnSO₄ x H₂O, 0.005 g L⁻¹ CuSO₄ x 5 H₂O, 0.4 g L⁻¹ EDTA); the pH was adjusted to 6.0 with 1 M NaOH prior to autoclaving. For SNL-agar plates, 15 g L⁻¹ agar was added to SNL.

Starter cultures were grown on 10 % (v/v) SNL with 5 % (w/v) wheat bran. Main cultures of U. *maydis* were cultivated in 10 % (v/v) SNL with 1 % (w/v) wheat bran as inductor for esterase production (26). After thirteen days of cultivation at 24 °C and 150 rpm on a rotary shaker (Infors, Bottmingen, Switzerland), cultures were harvested and stored until further processing at -20 °C.

### Enzyme purification

Culture supernatant of *U. maydis* was separated from the frozen cells by centrifugation (9.600 x g, 30 min, 4 °C) followed by filtration under reduced pressure using a 0.45 µm polyethersulfone (PES) membrane (Merck Millipore, Billerica, USA). 0.75 L filtered supernatant was diluted one time with buffer A (50 mM Bis-Tris pH 6.5) and applied to a 25 mL self-casted Q Sepharose FF column (GE Healthcare, Buckinghamshire, England) pre-equilibrated with buffer A. Fractions of 6.0 mL were collected at a flow rate of 4 mL min⁻¹ and monitored for esterase activity. Active fractions eluted with 50 % buffer B (buffer A with 1 M NaCl) were pooled, concentrated and desalted using an ultrafiltration module (30 kDa cut off, PES, Sartorius, Göttingen, Germany). Afterwards, a preparative isoelectric focusing (IEF) was performed with a Rotofor preparative IEF cell (Biorad, Hercules, USA). The concentrated, desalted sample was mixed with 2 % ampholyte (pH 2 to 4, Serva, Heidelberg, Germany). Two percent Chaps and proline were added to the sample to reduce protein precipitation during the run. The solution was focused at 12 W constant power for four hours at 4 °C. Fractions were collected, and the pH and esterase activity were determined. Active fractions from the preparative IEF were pooled and washed with an ultrafiltration module (30 kDa cut off, PES, Sartorius, Göttingen, Germany), to remove additives. Final purification of the enzyme was achieved with a combination of affinity and hydrophobic interaction chromatography using a 15 mL self-casted polyamide column (MP Biomedicals, Eschwege, Germany). The active sample was diluted one time with buffer (50 mM acetate pH 6.5 with 4 M (NH₄)₂SO₄) and loaded on the column. UmChIE was eluted with 65 % of 50 mM acetate buffer pH 6.5.

### Enzyme assay

Chlorogenic acid esterase activity was determined at 37 °C in 50 mM Tris buffer at pH 7.5 using chlorogenic acid and methyl ferulate as standard substrates, unless otherwise stated. The release of the corresponding free acids was analyzed with HPLC-UV. If appropriate, the hydrolysis of other benzoic and cinnamic acid ester derivatives as well as feruloylated saccharides was measured with the same method. After incubation for one hour the reaction was terminated by doubling the reacting volume with acetonitrile. Samples were centrifuged (15 min, 15.000 x g) before injection to the HPLC (Chromolith Performance RP-18e reverse-phase column, 100 x 4.6 mm, Merck, Darmstadt, Germany). Substrates and products were separated using a step-wise gradient at a flow velocity of 1.5 mL min⁻¹: 10 µL sample loaded in 90 % buffer A (0.1 % formic acid), 10 % buffer B (acetonitrile); 10 - 36 % buffer B in 8 min; 36 - 50 % buffer B in 1.5 min; 55 - 96 % buffer B in 0.5 min; 96 - 99 % buffer B in 2.5 min; 99 - 15 % buffer B in 0.5 min; 15 - 10 % buffer B in 1 min; re-equilibrate with 10 % buffer B for 1 min; total run time 14 min. Substance elution was detected at 232 nm (benzoic and 3-hydroxybenzoic acid), 254 nm (4-hydroxybenzoic and vanillic acid), 275 nm (cinnamic and gallic acid), 306 nm (*p*-coumaric acid) and 323 nm (ferulic, caffeic, chlorogenic and sinapic acid) using a Shimadzu UV-Vis detector (SPD-10A VP, Shimadzu Deutschland GmbH, Duisburg, Germany). One unit of enzyme activity was defined as the amount of enzyme releasing one µmol of free acid per minute under the specified conditions. All measurements were performed at least twice.

### Biochemical characterization of UmChIE

### i. Determination of temperature and pH optimum

The optimal pH was determined by measuring the esterase activity at 37 °C with pH in the range of 2 - 10 using the following buffers: 50 mM solutions of glycine (pH 2 - 3.5; pH 9 - 10), acetate (pH 3.5 - 5.5), Bis-Tris (pH 5.5 - 7), and Tris (pH 7 - 9). For the determination of the temperature optimum the activity assay was performed for 60 min at various temperatures (20 °C - 90 °C).

### ii. Temperature and pH stability

For determination of temperature stability the enzyme solution was incubated for 60 min at different temperatures (20 °C - 60 °C). Subsequently, the substrate and 50 mM Tris buffer pH 7.5 was added and the activity assay was performed for 60 min at 37 °C. The pH stability was determined using the above described buffer sytems (30 mM, pH 2 - 10) with incubation for 60 min at 37 °C. Residual enzyme activity was analyzed after further incubation (60 min, 37°C) in presence of substrate and 125 mM Tris buffer pH 7.5.

### iii. Effect of solvents, metal ions and inhibitors on enzyme activity

Enzyme tolerance to different solvents was investigated in the presence of 50 % (v/v) acetone, acetonitrile, diethyl ether, DMF, DMSO, ethanol, isoamyl alcohol, isobutyl alcohol, isopropanol, methanol, n-hexane, n-heptane, *tert*-butyl methyl ether or toluene, respectively, in 50 mM Tris buffer pH 7.5 containing 0.7 mM substrate. After incubation (60 min, 37 °C, 900 rpm) the mixture was applied for HPLC analysis as described above. The effect of inhibitors on enzyme activity was tested with following: cetyltrimethylammonium bromide (CTAB), EDTA, iodacetamide, phenyl-methylsulfonylfluoride (PMSF), SDS and various metal ions, such as Cs⁺, K⁺, Li⁺, Sr⁺, Ca²⁺, Co²⁺, Cu²⁺, Fe²⁺, Hg²⁺, Mg²⁺, Mn²⁺, Ni²⁺, Zn²⁺, Al³⁺, Fe³⁺ and Cr³⁺. Apart from PMSF (1 mM), all other potential inhibitors were added at concentrations of 5 mM. The effect of high salt concentrations (0.1 - 3 M NaCl) on enzyme activity was investigated, too.

### iv. Substrate specificity and kinetic parameters

Substrate specificity of the UmChIE was determined towards different cinnamate and benzoate esters as well as natural substrates, such as F-A, F-AX and F-AXG, with activity assays as described above. Three mM stock solutions of all substrates were prepared in water.

Kinetic parameters were determined from the initial hydrolysis rate of 0.005 - 1 mM chlorogenic acid, methyl ferulate and methyl *p*-coumarate (60 min, 37 °C, pH 7.5). *Kₘ* and vₘₐₓ values were calculated with non-linear regression using SigmaPlot 10.0 (Systat Software Inc., Chicago, USA). Blanks were carried out for all assays using water instead of enzyme. For calculation of *k*_{cat} the molecular mass was determined using denaturing SDS-PAGE.

### v. Enzymatic hydrolysis of natural substrates

Destarched wheat bran (DSWB) was prepared as described by Johnson et al. (27). Total content of ferulic, *p*-coumaric and caffeic acid in DSWB, sugar beet pectin (SBP) and coffee pulp was determined after alkaline hydrolysis with 2 M NaOH (4 h, 50 °C, 220 rpm). Phenolic acid content of each sample was analyzed with HPLC after acidification with acetic acid. The level of chlorogenic acid in coffee was determined after incubation with 80 % methanol. Samples (20 mg) of DSWB and coffee pulp as well as 0.5 % SBP were incubated (37°C, 20 h, 650 rpm) with purified UmChIE (0.02 U) in a final volume of 400 µL containing 125 mM Tris buffer pH 7.5. To investigate synergism with other carbohydrases, the release of ferulic acid was also analyzed after parallel incubation of UmChIE with either *Trichoderma viride* xylanase (DSWB, 0.025 U, Fluka, Buchs, Switzerland) or tomato pectin esterase (SBP, Sigma, Taufkirchen, Germany). The amount of released phenolic acids from the natural substrates was quantified using HPLC.

### Denaturing SDS-PAGE

The enzyme purity and the molecular mass was determined using SDS-PAGE analysis (12 % resolving gel, 4 % stacking gel) according to Laemmli (28). Samples were prepared by mixing 1:1 with sample buffer (0.1 M Tris-HCl pH 6.8, 0.2 M DTT, 4 % SDS, 20 % glycerol, 0.2 % bromophenol blue) and boiling for 10 min. After electrophoresis at 15 mA per gel, gels were stained with InstantBlue (Expedeon, Cambridgeshire, Great Britain).

### Peptide mass fingerprint

Amino acid sequences of tryptic peptides of the UmChIE were deduced from ESI-MS/MS using a maXis QTOF mass spectrometer (Bruker, Bremen, Germany). The procedure is described in detail elsewhere (25). NCBI BLAST (program blastp) searches were performed with the obtained amino acid sequences.

### Amplification of chlorogenic acid esterase sequence

Isolation of total RNA from mycelium of *U. maydis,* grown for thirteen days in 10 % (v/v) SNL supplemented with 1 % (w/v) wheat bran, as well as cDNA synthesis were performed as described by Linke et al. (25). Amplification of the complete *CHLE* sequence was achieved by using the untranslated region (UTR) primers Uma_fw_UTR (TCCGTTCTTAGAAGCCAAACA (SEQ ID NO: 5)) and Uma_rev_UTR (GAAAGCCAAGCAACAAGGTT (SEQ ID NO: 6)), which were deduced based upon available Um00182.1 gene sequence (GenBank accession no. XP_756329.1). PCRs were performed by mixing 10 µL 5x Phusion High-Fidelity DNA Polymerase buffer, 0.4 µL dNTP mix (10 mM each), 25 pmol forward primer, 25 pmol reverse primer, 0.02 U Phusion High-Fidelity DNA Polymerase (Fermentas, St. Leon-Roth, Germany), 100 ng ss cDNA, and ddH₂O to 50 µL. PCR was performed in a MasterCycler gradient (Eppendorf, Hamburg, Germany) by incubating the reaction mixture at 98 °C for 2 min, then for 35 cycles at 98 °C for 30 s, 60 °C for 30 s and 72 °C for 60 s. Final elongation was performed at 72 °C for 10 min. All further steps including analysis of PCR products, ligation, transformation in *E. coli* and colony PCR were performed as described previously (29).

### Heterologous expression in Pichia pastoris

The *CHLE* gene with and without the native 21 amino acid signal peptide sequence was amplified using the primers UmChIE_Sfw (TTT TTC TCG AGA AAA GAG AGG CTA GGC TGC CTA ATC TG (SEQ ID NO: 7)), UmChIE_Ofw (TTT TTC TCG AGA AAA GAG AGG CTG AAG CTC TTC CAC AAG TCT C (SEQ ID NO: 8)) and UmChIE_rv (TTT TTG CGG CCG CTT AAT GAT GAT GAT GAT GAT GGA AGC CAA ACA C (SEQ ID NO: 9)) (underlined bases are the *Xho*I and *Not*I restriction sites, respectively) and inserted into the *Xho*I*-Not*I site of the P. *pastoris* expression vector pPIC9 (Invitrogen, Karlsruhe, Germany). P. *pastoris* GS115 was transformed with the *Pme*I linearized expression construct pPIC9*-HIS-CHLE* to achieve *CHLE* gene insertion at the *AOX1* locus by electroporation (MicroPulser Electroporator, Bio-Rad, München, Germany) (30). After selection of P. *pastoris* transformants with the ability to grow on histidine-deficient agar plates, 48 transformants of each expression construct were screened for chlorogenic acid esterase activity. The transformants were cultivated in 96-well-plates as described by Sygmund et al. (31). Culture supernatants were analyzed for enzyme activity after 24, 48, 72, 96 and 120 h with the assay described above. To maintain stable expression conditions 0.5 % (v/v) methanol was added daily.

### Results and discussion

### Purification and general properties of UmChIE

When *U. maydis* was cultivated in SNL medium, esterase activity was detected, but the cultures turned deep black impeding chromatographic purification. The composition of SNL medium seemed to promote the synthesis of melanins. Melanin production has been described for U. *maydis* (32, 33). Production was avoided by reducing SNL to 10 % (v/v) and adding 1 % (w/v) wheat bran. These conditions induced esterase production with even higher activities than in the black SNL cultures. After optimization of the culture medium, the purification of the chlorogenic acid esterase UmChIE from culture supernatant of *U. maydis* was accomplished by three complementary separation steps including anion exchange chromatography on Q Sepharose FF, preparative IEF and finally a combination of affinity and hydrophobic interaction chromatography on polyamide.

The molecular mass of the chlorogenic acid esterase as determined by denaturing SDS-PAGE was 71 kDa (Fig.1, lane 2). The apparent mass of the native enzyme estimated by size exclusion chromatography suggested that the UmChIE was a monomer. These results demonstrated that UmChIE differed from the other enzymes described in literature. While Asther et al. (13) and Adachi et al. (15) identified the native chlorogenic acid hydrolases as homodimers of about 2 x 80 kDa, Benoit et al. (14) suggested the recombinant *A. niger* enzyme to exist as tetrameric form. Schöbel and Pollmann (12) characterized the second known chlorogenic acid esterase from A. *niger* also as a tetramer (4 x 60 kDa), whereas Okamura and Watanabe (16) described the hydroxycinnamic acid ester hydrolase of *A. japonicus* as a monomer of 145 kDa. The preparative IEF, used as second purification step, yielded a pl of about 3.0 for the UmChIE.

### Characterization of the purified chlorgenic acid esterase

To date, only few chlorogenic acid hydrolases (EC 3.1.1.42) are well characterized (11-15), while numerous feruloyl esterases (FAE; EC 3.1.1.73), a subclass of the carboxylic ester hydrolases, were purified and characterized from a wide range of bacteria and fungi (7-10). Some feruloyl esterases showed the ability to hydrolyze chlorogenic acid, such as *A. niger* FAEB (34) and a type B feruloyl esterase from *Neurospora crassa* (35). As UmChIE accepted chlorgenic acid as the most preferred substrate, but also hydrolyzed feruloyl esterase substrates efficiently, the enzyme was compared both with chlorogenic acid hydrolases and feruloyl esterases in terms of their properties.

As shown in Fig. 2, the influence of pH on esterase activity and stability was investigated in the pH range of 2 - 10. Maximum activity for hydrolysis of methyl ferulate was observed at pH 7.5, and more than 50 % residual activity was detected in the pH range of 5.5 - 9.5 (Fig. 2A). After one hour incubation at 37 °C the enzyme was more stable in neutral to alkaline than in acidic conditions (Fig. 2B). UmChIE retained more than 80 % of its maximum activity between pH 4.5 - 10. The determined pH optima of the two published chlorogenic acid hydrolases from *A. niger* (11, 13) and of type B FAEs listed by Koseki et al. (36) were between pH 6 and 7. Especially the pH stability of UmChIE was different from the characterized chlorogenic acid hydrolases from *A. niger* which were more stable in acidic than in alkaline conditions. The chlorogenic acid esterase isolated by Schöbel and Pollmann (12) was stable in a pH range of 3 - 8.5 after 10 minutes of incubation at an undefined temperature. Thus, UmChIE has a significant pH stability of the in alkaline conditions, which makes the enzyme an attractive candidate for biotechnological applications, for instance, the treatment of pulp in the paper industry (37).

Optimal reaction temperature of the UmChIE was recorded at 37 °C (Fig. 2C). Enzyme activity started to decrease rapidly at 45 °C to be nearly inactive at 60 °C. After pre-incubation at pH 7.5 and 37 °C for one hour, the enzyme lost 28 % of its activity. The thermostability of UmChIE decreased rapidly above 40 °C. The determined temperature optimum was lower than those reported for chlorogenic acid esterases and most of the feruloyl esterases (36). Furthermore the purified enzyme was less thermostable than those previously described (12-14).

The applicability of the UmChIE for ester synthesis was examined in 1:1 (v/v) buffer emulsions with different organic solvents (see Table 1). Relative enzyme activities were calculated based on a sample incubated in buffer without organic solvent (37 °C; one hour, pH 7.5, 900 rpm). Some of the tested solvents are water-immiscible, but a large interfacial area between organic and polar phase was ensured by vigorous shaking of the reaction mixture. UmChIE showed good stability in *n-*hexane and n-heptane with relative activities of 87 % and 84 %. Moderate to slight activity was detected in isoamyl alcohol (64 %) and isobutyl alcohol (23 %). In all other systems the residual enzyme activity was less than 7 %. The good stability of the UmChIE in n-hexane and n-heptane indicated the potential of the enzyme for reverse-synthetic activity. Indeed, Topakas et al. showed the synthesis of phenolic acid esters with a feruloyl esterase of *Fusarium oxysporum* (38) and *Sporotrichum thermophile* (39) using a surfactantless microemulsion formed in a ternary water-organic mixture consisting of n-hexane, 1-propanol/1-butanol and water as a reaction system.

**Table 1 Effect of organic solvents on UmChIE activity.**

| **Organic solvent** | **Relative esterase activity [%]** | |
|---|---|---|
| *n*-hexane | 87 | (2.12) |
| *n*-heptane | 84 | (0.89) |
| isoamyl alcohol | 64 | (2.43) |
| isobutyl alcohol | 23 | (6.53) |
| Toluene | 7 | (1.67) |
| Isopropanol | 6 | (0.37) |
| Methanol | 5 | (0.23) |
| Acetone | 3 | (2.59) |
| acetonitrile | 3 | (3.18) |
| DMF | 1 | (0.76) |
| diethyl ether | ND | |
| DMSO | ND | |
| Ethanol | ND | |
| *tert*-butyl methyl ether | ND | |

Residual activities of UmChIE for the hydrolysis of 0.7 mM methyl ferulate (1 h, 37 °C, 900 rpm) in a binary system composed of Tris buffer pH 7.5 and organic solvents in the ratio 1:1 (v/v). Esterase activity determined without organic solvent was set to 100 %. Numbers in parentheses are the estimates of the standard error. ND: activity not detectable.

### Inhibition studies

Various metal ions and enzyme inhibitors were examined for their effect on chlorogenic acid esterase activity (Table 2). Residual enzyme activity was measured using the standard activity assay in presence of 5 mM metal ions/inhibitors (except PMSF, 1 mM). Addition of Hg²⁺ resulted in complete loss of enzyme activity. Hg²⁺ reacts with thiol groups and can also reduce disulphide bonds (40). Among the tested metal ions, a significant inhibition of UmChIE activity, to residual activities of 50 - 76 %, was detected in presence of Al³⁺, Co²⁺, Cr³⁺, Fe³⁺ and Zn²⁺, while Mn²⁺ decreased the enzyme activity slightly to 90 %. Accordingly, the enzyme of the invention can be used in the presence of all tested metal ions except Hg²⁺ with significant residual activity. The inhibition of UmChIE activity by Zn²⁺, Hg²⁺ and Al³⁺ was consistent with the properties of a feruloyl esterase from *Russula virescens* (41). This feruloyl esterase was also inhibited by Fe²⁺, whereas UmChIE was not, but it was only inhibited by Fe³⁺. The negative effect of Fe³⁺ on esterase activity was also shown by Donaghy and McKay (42) and Yao et al. (43). Some esterases were also inhibited by Cu²⁺ (42, 43) and Cd²⁺ (41).

**Table 2 Effect of metal ions, inhibitors and NaCl on UmChIE activity.**

| **Concentration [mM]** | **Substance** | **Relative esterase activity [%]** | |
|---|---|---|---|
| 5 | KCI | 108 | (0.57) |
| 5 | NiSO₄ | 108 | (0.87) |
| 5 | MgCl₂ | 106 | (0.53) |
| 5 | LiCl | 106 | (1.39) |
| 5 | CsCl | 105 | (1.21) |
| 5 | SrNO₃ | 104 | (0,18) |
| 5 | CaCl₂ | 103 | (2.76) |
| 5 | FeSO₄ | 102 | (0.50) |
| 5 | Cu(NO₃)₂ | 96 | (0.53) |
| 5 | MnSO₄ | 90 | (0.09) |
| 5 | ZnSO₄ | 76 | (1.26) |
| 5 | Cr(NO₃)₃ | 69 | (3.54) |
| 5 | CoCl₂ | 67 | (0.11) |
| 5 | FeCl₃ | 66 | (0. 65) |
| 5 | Al₂(SO₄)₃ | 53 | (0.12) |
| 5 | HgAc₂ | ND | |
| 5 | EDTA | 105 | (1.14) |
| 5 | CTAB | 104 | (0.70) |
| 5 | lodacetamide | 102 | (0.81) |
| 5 | SDS | 59 | (0.83) |
| 1 | PMSF | 36 | (2.01) |
| 100 | NaCl | 105 | (0.02) |
| 500 | NaCl | 121 | (0.01) |
| 1000 | NaCl | 125 | (0.02) |
| 2000 | NaCl | 142 | (0.06) |
| 3000 | NaCl | 150 | (0.04) |

Remaining enzyme activity was measured after 1 h incubation with 1 mM methyl ferulate and different metal ions as well as inhibitors in concentrations listed above (at 37 °C, 50 mM Tris buffer pH 7.5). Numbers in parentheses are the estimates of the standard error. A sample without additive was used for the calculation of relative enzyme activities. ND: activity not detectable.

The enzyme activity was inhibited 41 % by SDS and 64 % by PMSF, while incubation with CTAB, EDTA and iodacetamide showed no effect (Table 2). The almost complete loss of activity in the presence of PMSF, a known esterase inhibitor, suggested that the catalytic triad of UmChIE contained a serine residue (44). In contrast, enzyme activity was not influenced by CTAB, which reacts with tryptophane and SH groups of cysteine, indicating that these amino acid residues were not involved in enzyme catalysis (45).

Furthermore, the tolerance of UmChIE towards various NaCl concentrations (0.1 - 3 M) was investigated. It was shown that the enzyme activity increased with the salt concentration, up to 150 % in presence of 3 M NaCl (Table 2). A stimulatory effect on esterase activity by NaCl was previously reported by Nieter et al. (29) and Kumar et al. (46).

### Hydrolytic specificity and kinetic properties of UmChIE

A comprehensive set of substrates was examined for hydrolysis by UmChIE. The substrates included cinnamic- and benzoic acid esters as well as natural substrates, such as F-A, F-AX, F-AXG and chlorogenic acid (Table 3). Chlorgenic acid was found to be hydrolyzed most effectively. The determined specific activity of 2.8 U mg⁻¹ for chlorogenic acid was set to 100 % and used for the calculation of relative activities for all other substrates. UmChIE hydrolyzed methyl p-coumarate (M*p*CA, 52 %), methyl ferulate (MFA, 46 %) and methyl caffeate (MCA, 18 %), but no methyl sinapate (MSA). These four typical synthetic feruloyl ester substrates were commonly used to classify FAEs. Crepin et al. (10) divided FAEs into four types (A - D) based on their substrate specificity, sequence homology and biochemical analysis. The lack of hydrolysis of MSA would result in type B FAE according to Crepin et al. (10). In the meantime the classification system was extended by phylogenetic analysis of fungal FAEs by Benoit et al. (47), while a descriptor-based computational analysis with pharmacophore modeling (48) offered an alternative attempt for classification.

**Table 3 Substrate specificity of purified UmChIE.**

| **Substance** | **Relative esterase activity [%]** | | **Specific esterase activity [U mg⁻¹]** |
|---|---|---|---|
| chlorogenic acid | 100 | | 2.76 |
| F-A | 75 | (0.16) | 2.06 |
| F-AX | 59 | (0.36) | 1.63 |
| methyl p-coumarate | 52 | (0.06) | 1.43 |
| methyl ferulate | 46 | (0.16) | 1.27 |
| F-AXG | 33 | (0.09) | 0.92 |
| ethyl ferulate | 28 | (0.45) | 0.78 |
| methyl caffeate | 18 | (0.03) | 0.49 |
| methyl benzoate | ND | | ND |
| ethyl benzoate | ND | | ND |
| methyl 3-hydroxy benzoate | ND | | ND |
| methyl 4-hydroxy benzoate | ND | | ND |
| ethyl 4-hydroxy benzoate | ND | | ND |
| propyl 4-hydroxy benzoate | ND | | ND |
| butyl 4-hydroxy benzoate | ND | | ND |
| methyl cinnamate | ND | | ND |
| ethyl cinnamate | ND | | ND |
| methyl gallate | ND | | ND |
| methyl sinapate | ND | | ND |
| methyl vanillate | ND | | ND |
| ethyl vanillate | ND | | ND |

Purified UmChIE was incubated with 1 mM of different substrates for 1 h (at 37 °C, 50 mM Tris buffer pH 7.5). Relative enzyme activities were calculated based on the most preferred substance of the UmChIE: chlorogenic acid which was set to 100 %. Numbers in parentheses are the estimates of the standard error.
Abbreviations: F-A: 5-O-*trans*-feruloyl-L-arabinofuranose, F-AX: β-D-xylopyranosyl-(1→2)-5-O-*trans*-feruloyl-L-arabinofuranose, F-AXG: α-L-galactopyranosyl-(1→2)-β-D-xylopyranosyl-(1→2)-5-O-*trans*-feruloyl-L-arabinofuranose and ND: activity not detectable.

UmChIE was also able to release ferulic acid from ethyl ferulate, but about 50 % less than from MFA. Consequently, elongation of the ester-linked alkyl chain resulted in decreasing enzyme activity (49). While the presence of one methoxy group at the *meta* position of the benzoic ring lowered enzyme activity marginally (M*p*CA 52 % vs. MFA 46 %), a second methoxy group caused total loss of activity (MSA). Moreover UmChIE hydrolyzed no substrate without hydroxyl or methoxy group on the benzoic ring (methyl/ethyl cinnamate). The fact, that UmChIE showed no hydrolytic activity against any benzoate derivative indicates the necessity of a certain distance between the aromatic ring and the ester bond. This requirement for the catalytic activity of feruloyl esterases was also reported by Topakas et al. (50) and Kroon et al. (51).

UmChIE hydrolyzed also small natural substrates such as feruloylated mono-, di- and trisaccharides (F-A, F-AX, F-AXG). F-A was hydrolyzed most efficiently (2.0 U mg⁻¹) followed by FAX (1.6 U mg⁻¹) and F-AXG (0.9 U mg⁻¹). Likewise, *A. niger* FAEA (34) preferred the natural FAE substrates in the same order, whereas PeFaeA of *Pleurotus eryngii* (29) and Est1 of *Pleurotus sapidus* (25) preferred the natural substrate F-A less than F-AX. In contrast, the FAE of *Penicillium expansum* had higher specific activities for methyl ferulate (14.4 U mg⁻¹) than for F-AX (5.3 U mg⁻¹) (42).

Kinetic properties were determined from initial hydrolysis rates for the most preferred substrates chlorogenic acid, MFA and M*p*CA. The purified UmChIE possessed the following kinetic constants (*K*ₘ; *k*_{cat}; *k*_{cat}/*K*ₘ): MFA (101.8 µM; 0.38 s⁻¹; 3.75 mM⁻¹s⁻¹), M*p*CA (64.1 µM; 0.49 s⁻¹; 7.63 mM⁻¹s⁻¹) and chlorogenic acid (19.6 µM; 0.51 s⁻¹; 25.83 mM⁻¹s⁻¹). Determination of *K*ₘ values demonstrated that the enzyme had a three- and fivefold higher affinity for chlorogenic acid than for M*p*CA and MFA. Furthermore the *k*_{cat}/*K*ₘ values revealed that the UmChIE hydrolyzed chlorogenic acid three-and sevenfold more efficiently than M*p*CA and MFA. Consequently, the catalytic efficiency (*k*_{cat}/*K*ₘ), a good indicator for enzyme efficiency and specificity, was better for chlorogenic acid than for the two synthetic substrates.

While the UmChIE could still be classified as a feruloyl esterase based on its substrate profile, the kinetic constants clearly showed the affiliation to the family of chlorogenic acid esterases. Here, the previously reported *K*ₘ values for chlorogenic acid as substrate were 6.5 - 10 µM (13, 14) and 0.7 mM (11) for the second published chlorogenic acid hydrolases from *A. niger.* There is few other literature available dealing with an enzyme activity on chlorogenic acid. In contrast, *K*ₘ values were determined for three type B feruloyl esterases for the substrate chlorogenic acid as following: 245 µM for *A. niger* FAEB (34), 180 µM for type B feruloyl esterase from *N. crassa* (35) and 630 µM for AoFaeB from *A. oryzae* (19). The feruloyl esterases mentioned above exhibited not only a more than tenfold lower affinity for chlorogenic acid than UmChIE but possessed also a threefold lower *K*m for M*p*CA (20 µM) than UmChIE (64 µM). They also showed higher specificity for MCA than for MFA.

Finally, the UmChIE distinguished clearly from the two other published enzymes by its broad substrate profile. Previously identified chlorogenic acid hydrolases were solely active on chlorogenic acid. Asther et al. (13) tested activity of the native chlorogenic acid hydrolase for methyl ester of hydroxycinnamates, while Benoit et al. (14) investigated F-A and F-AX as substrates for the recombinant enzyme, but none of these substrates was hydrolyzed.

### Release of phenolic acids from natural substrates

For the determination of UmChIE specificity, SBP and DSWB were used as substrates due to the different linkage of ferulic acid to arabinose residues (3). The agro-industrial by-product coffee pulp was selected due to its high content of chlorogenic acid. Total amounts of caffeic acid, ferulic and p-coumaric acid were quantified after alkaline hydrolysis with HPLC for coffee pulp (1.65 mg g⁻¹, 0.53 mg g⁻¹, 0.07 mg g⁻¹), DSWB (- ; 4.45 mg g⁻¹; 0.18 mg g⁻¹) and SBP (- ; 6.78 mg g⁻¹; - ;). Further, coffee pulp contained 2.66 mg g⁻¹ of chlorogenic acid in total.

After incubation of coffee pulp with UmChIE (0.02 U; 20 h, 37 °C and 650 min⁻¹ on a thermoshaker) 68 % of the initial chlorogenic acid amount was hydrolyzed. In contrast, the native as well as the recombinant chlorogenic acid hydrolase from *A. niger* released after overnight incubation 100 % of the initial chlorogenic acid content of coffee pulp, but the enzymes were used with two to sixfold higher concentrations than UmChIE (13, 14). UmChIE also liberated significant amounts of phenolic acids from coffee pulp. Thus, 18 %, 15 % and 26 % of the alkali-extractable caffeic, ferulic and p-coumaric acid level were released, respectively. The higher content of alkali-extractable caffeic acid than the total amount of chlorogenic acid can be explained by the existence of caffeic acid conjugates other than chlorogenic acid, such as dicaffeoyl quinic acid or esters of feruloyl-caffeoyl quinic acid (4).

While the UmChIE (0.02 U) liberated no ferulic acid from SBP, a small release was observed from DSWB. The simultaneous incubation with *T. viride* xylanase (0.025 U) increased the amount of released ferulic acid from DSWB more than twelvefold, whereas the incubation with *T. viride* xylanase liberated no ferulic acid at all. The observed synergistic action of xylanases and feruloyl esterases was reported previously (26, 52-54). This result can be explained by the action of endoxylanases producing shorter-chain xylo-oligosaccharides which are more accessible substrates for esterases. The lack of release of ferulic acid from SBP indicated that UmChIE was not active on O-2 of arabinose or O-6 ester linkages of galactose. Benoit et al. (34) compared the *A. nigerferuloyl* esterases FAEA and FAEB in terms of their activity on wheat bran and SBP. FAEA was mainly active on O-5 esters of arabinose present in wheat bran and FAEB on O-2 or O-6 ester linkages. Although the substrate specificity of UmChIE indicated a feruloyl esterase type B side activity, it was not active on SBP.

### Amplification and genetic characterization of the UmChIE sequence

Seven tryptic peptides of the purified chlorogenic acid esterase were obtained by ESI-MS/MS: ADATASAPTVK (P1, SEQ ID NO: 10), FAKPQPLGPASSHK (P2, SEQ ID NO: 11), HPTVEQSFKR (P3, SEQ ID NO: 12), LANGVGCTGGSLLR (P4, SEQ ID NO: 13), VWSYEFQQNDK (P5, SEQ ID NO: 14), EAMDALTNR (P6, SEQ ID NO: 15) and AKPPVGSLR (P7, SEQ ID NO: 16). Homology search with Blastp (NCBI BLAST) identified them (P1 - P7) as part of the hypothetical protein Um00182.1 from *U. maydis* 521 (GenBank accession no. **XP_756329.1)**. After RNA isolation from *U. maydis* cultures grown for thirteen days in 10 % (v/v) SNL media supplemented with 1 % (w/v) wheat bran, cDNA was synthesized, and the sequence of the UmChIE (GenBank accession no. **HG970190)** was successfully amplified using UTR primers. The complete coding sequence of UmChIE has a length of 1,758 bp, corresponding to a protein of 585 amino acids (aa). The full nucleic acid sequence of UmChIE (SEQ ID NO: 18) possessed three differences on nucleotide level which led to one change on the amino acid level (SEQ ID NO: 17) at position 492 (proline to leucine) compared to the sequence of Um00182.1 (SEQ ID NO: 19).

### Heterologous expression of UmChIE in P. pastoris

To confirm that the annotated sequence (GenBank accession no. HG970190) encodes the characterized chlorogenic acid esterase UmChIE from *U. maydis,* the putative sequence was cloned in frame with the *Saccharomyces cerevisiae* α-factor secretion signal sequence under the transcriptional control of the alcohol oxidase *(AOX1)* promoter and integrated into *P. pastoris* GS115. Expression constructs were generated with and without the native signal sequence. A hexahistidine tag was incorporated at the C-terminus. Only the transformants carrying the expression construct without native signal sequence showed chlorogenic acid esterase activity. After induction with 0.5 % (v/v) methanol, these *P. pastoris* transformants expressed the recombinant gene successfully and secreted the active enzyme into the culture media. After methanol induction for 96 h the chlorogenic acid esterase activity of the culture medium (0.8 U L⁻¹) was eightfold higher than in control cultures of *P*. *pastoris* GS115.

### REFERENCES

1. Ishii T. 1997. Structure and function of feruloylated polysaccharides. Plant Sci. 127:111-127.
2. Cosgrove DJ. 2001. Wall structure and wall loosening. A look backwards and forwards. Plant physiology 125:131-134.
3. Ralet MC, Faulds CB, Williamson G, Thibault JF. 1994. Degradation of feruloylated oligosaccharides from sugar-beet pulp and wheat bran by ferulic acid esterases from Aspergillus niger. Carbohydrate research 263:257-269.
4. Clifford MN. 1999. Chlorogenic acids and other cinnamates - nature, occurrence, dietary burden, absorption and metabolism. J Sci Food Agric 79:362-372.
5. Kroon PA, Garcia-Conesa MT, Fillingham IJ, Hazlewood GP, Williamson G. 1999. Release of ferulic acid dehydrodimers from plant cell walls by feruloyl esterases. J Sci Food Agric 79:428-434.
6. Williamson G, Faulds CB, Kroon PA. 1998. Specificity of ferulic acid (feruloyl) esterases. Biochemical Society transactions 26:205-209.
7. Wong DW. 2006. Feruloyl esterase: a key enzyme in biomass degradation. Applied biochemistry and biotechnology 133:87-112.
8. Fazary AE, Ju YH. 2007. Feruloyl esterases as biotechnological tools: current and future perspectives. Acta biochimica et biophysica Sinica 39:811-828.
9. Topakas E, Vafiadi C, Christakopoulos P. 2007. Microbial production, characterization and applications of feruloyl esterases. Process Biochem. 42:497-509.
10. Crepin VF, Faulds CB, Connerton IF. 2004. Functional classification of the microbial feruloyl esterases. Applied Microbiology and Biotechnology 63:647-652.
11. Schöbel B, Pollmann W. 1980. Further characterization of a chlorogenic acid hydrolase from Aspergillus niger. Zeitschrift fur Naturforschung. Section C: Biosciences 35:699-701.
12. Schöbel B, Pollmann W. 1980. Isolation and characterization of a chlorogenic acid esterase from Aspergillus niger. Zeitschrift fur Naturforschung. Section C: Biosciences 35:209-212.
13. Asther M, Estrada Alvarado MI, Haon M, Navarro D, Asther M, Lesage-Meessen L, Record E. 2005. Purification and characterization of a chlorogenic acid hydrolase from Aspergillus niger catalysing the hydrolysis of chlorogenic acid. Journal of biotechnology 115:47-56.
14. Benoit I, Asther M, Bourne Y, Navarro D, Canaan S, Lesage-Meessen L, Herweijer M, Coutinho PM, Asther M, Record E. 2007. Gene overexpression and biochemical characterization of the biotechnologically relevant chlorogenic acid hydrolase from Aspergillus niger. Applied and environmental microbiology 73:5624-5632.
15. Adachi O, Ano Y, Akakabe Y, Shinagawa E, Matsushita K. 2008. Coffee pulp koji of Aspergillus sojae as stable immobilized catalyst of chlorogenate hydrolase. Applied Microbiology and Biotechnology 81:143-151.
16. Okamura S, Watanabe M. 1982. Purification and properties of hydroxycinnamic acid ester hydrolase from Aspergillus japonicus. Agric. Biol. Chem. 46:1839-1848.
17. Barbe C, Dubourdieu D. 1998. Characterization and purification of a cinnamate esterase from Aspergillus niger industrial pectinase preparation. J Sci Food Agric 78:471-478.
18. Levasseur A, Benoit I, Asther M, Asther M, Record E. 2004. Homologous expression of the feruloyl esterase B gene from Aspergillus niger and characterization of the recombinant enzyme. Protein expression and purification 37:126-133.
19. Koseki T, Hori A, Seki S, Murayama T, Shiono Y. 2009. Characterization of two distinct feruloyl esterases, AoFaeB and AoFaeC, from Aspergillus oryzae. Applied Microbiology and Biotechnology 83:689-696.
20. Vafiadi C, Topakas E, Christakopoulos P, Faulds CB. 2006. The feruloyl esterase system of Talaromyces stipitatus: determining the hydrolytic and synthetic specificity of TsFaeC. Journal of biotechnology 125:210-221.
21. Qi M, Wang P, Selinger LB, Yanke LJ, Forster RJ, McAllister TA. 2011. Isolation and characterization of a ferulic acid esterase (Fae1A) from the rumen fungus Anaeromyces mucronatus. Journal of applied microbiology 110:1341-1350.
22. Couteau D, McCartney AL, Gibson GR, Williamson G, Faulds CB. 2001. Isolation and characterization of human colonic bacteria able to hydrolyse chlorogenic acid. Journal of applied microbiology 90:873-881.
23. Borneman WS, Hartley RD, Morrison WH, Akin DE, Ljungdahl LG. 1990. Feruloyl and p-coumaroyl esterase from anaerobic fungi in relation to plant cell wall degradation. Applied Microbiology and Biotechnology 33:345-351.
24. Allerdings E, Ralph J, Steinhart H, Bunzel M. 2006. Isolation and structural identification of complex feruloylated heteroxylan side-chains from maize bran. Phytochemistry (Elsevier) 67:1276-1286.
25. Linke D, Matthes R, Nimtz M, Zorn H, Bunzel M, Berger RG. 2013. An esterase from the basidiomycete Pleurotus sapidus hydrolyzes feruloylated saccharides. Applied Microbiology and Biotechnology 97:7241-7251.
26. Garcia-Conesa MT, Crepin VF, Goldson AJ, Williamson G, Cummings NJ, Connerton IF, Faulds CB, Kroon PA. 2004. The feruloyl esterase system of Talaromyces stipitatus: production of three discrete feruloyl esterases, including a novel enzyme, TsFaeC, with a broad substrate specificity. Journal of biotechnology 108:227-241.
27. Johnson KG, Harrison BA, Schneider H, MacKenzie CR, Fontana JD. 1988. Xylan-hydrolysing enzymes from Streptomyces spp. Enzyme Microb. Technol. 10**.**
28. Laemmli UK. 1970. Cleavage of structural proteins during the assembly of the head of bacteriophage T4. Nature 227:680-685.
29. Nieter A, Haase-Aschoff P, Linke D, Nimtz M, Berger RG. 2014. A halotolerant type A feruloyl esterase from Pleurotus eryngii. Fungal biology 118:348-357.
30. Lin-Cereghino J, Wong WW, Xiong S, Giang W, Luong LT, Vu J, Johnson SD, Lin-Cereghino GP. 2005. Condensed protocol for competent cell preparation and transformation of the methylotrophic yeast Pichia pastoris. BioTechniques 38:44, 46, 48.
31. Sygmund C, Gutmann A, Krondorfer I, Kujawa M, Glieder A, Pscheidt B, Haltrich D, Peterbauer C, KittI R. 2012. Simple and efficient expression of Agaricus meleagris pyranose dehydrogenase in Pichia pastoris. Applied Microbiology and Biotechnology 94:695-704.
32. Nicolaus RA, Piattelli M. 1965. Progress in the chemistry of natural black pigments. Academia delle Scienze Fisiche e Matematiche in Napoli. Rendiconto (Series 4) 32:83 - 97.
33. Wheeler MH. 1983. Comparison of fungal melanin biosynthesis in ascomycetous, imperfect and basidiomycetous fungi. Trans. Br. mycol. Soc. 81:29-36.
34. Benoit I, Navarro D, Marnet N, Rakotomanomana N, Lesage-Meessen L, Sigoillot JC, Asther M, Asther M. 2006. Feruloyl esterases as a tool for the release of phenolic compounds from agro-industrial by-products. Carbohydrate research 341:1820-1827.
35. Crepin VF, Faulds CB, Connerton IF. 2003. A non-modular type B feruloyl esterase from Neurospora crassa exhibits concentration-dependent substrate inhibition. The Biochemical journal 370:417-427.
36. Koseki T, Fushinobu S, Ardiansyah, Shirakawa H, Komai M. 2009. Occurrence, properties, and applications of feruloyl esterases. Applied Microbiology and Biotechnology 84:803-810.
37. Sigoillot C, Camarero S, Vidal T, Record E, Asther M, Perez-Boada M, Martinez MJ, Sigoillot JC, Colom JF, Martinez AT. 2005. Comparison of different fungal enzymes for bleaching high-quality paper pulps. Journal of biotechnology 115:333-343.
38. Topakas E, Stamatis H, Biely P, Kekos D, Macris BJ, Christakopoulos P. 2003. Purification and characterization of a feruloyl esterase from Fusarium oxysporum catalyzing esterification of phenolic acids in ternary water-organic solvent mixtures. Journal of biotechnology 102:33-44.
39. Topakas E, Stamatis H, Biely P, Christakopoulos P. 2004. Purification and characterization of a type B feruloyl esterase (StFAE-A) from the thermophilic fungus Sporotrichum thermophile. Applied Microbiology and Biotechnology 63:686-690.
40. Vallee BL, Ulmer DD. 1972. Biochemical effects of mercury, cadmium and lead. Annu Rev Biochem. 41:91-128.
41. Wang L, Zhang R, Ma Z, Wang H, Ng T. 2014. A feruloyl esterase (FAE) characterized by relatively high thermostability from the edible mushroom Russula virescens. Applied biochemistry and biotechnology 172:993-1003.
42. Donaghy J, McKay AM. 1997. Purification and characterization of a feruloyl esterase from the fungus Penicillium expansum. Journal of applied microbiology 83:718-726.
43. Yao J, Chen QL, Shen AX, Cao W, Liu YH. 2013. A novel feruloyl esterase from a soil metagenomic library with tannase activity. J. Mol. Catal. B: Enzym. 95:55-61.
44. Gold AM, Fahrney D. 1964. Sulfonyl fluorides as inhibitors of esterases. II. Formation and reactions of phenylmethanesulfonyl alpha chymotrypsin. Biochemistry 3:783-791.
45. Anson ML. 1940. The reactions of iodine and iodoacetamide with native egg albumin. The Journal of general physiology 23:321-331.
46. Kumar L, Singh B, Adhikari DK, Mukherjee J, Ghosh D. 2012. A thermoalkaliphilic halotolerant esterase from Rhodococcus sp. LKE-028 (MTCC 5562): Enzyme purification and characterization. Process Biochem. 47:983-991.
47. Benoit I, Danchin EGJ, Bleichrodt R-J, de Vries RP. 2008. Biotechnological applications and potential of fungal feruloyl esterases based on prevalence, classification and biochemical diversity. Biotechnology Letters 30:387-396.
48. Udatha DB, Kouskoumvekaki I, Olsson L, Panagiotou G. 2011. The interplay of descriptor-based computational analysis with pharmacophore modeling builds the basis for a novel classification scheme for feruloyl esterases. Biotechnology advances 29:94-110.
49. de Vries RP, vanKuyk PA, Kester HC, Visser J. 2002. The Aspergillus niger faeB gene encodes a second feruloyl esterase involved in pectin and xylan degradation and is specifically induced in the presence of aromatic compounds. The Biochemical journal 363:377-386.
50. Topakas E, Christakopoulos P, Faulds CB. 2005. Comparison of mesophilic and thermophilic feruloyl esterases: characterization of their substrate specificity for methyl phenylalkanoates. Journal of biotechnology 115:355-366.
51. Kroon PA, Faulds CB, Brezillon C, Williamson G. 1997. Methyl phenylalkanoates as substrates to probe the active sites of esterases. European journal of biochemistry / FEBS 248:245-251.
52. Faulds CB, Sancho AI, Bartolome B. 2002. Mono- and dimeric ferulic acid release from brewer's spent grain by fungal feruloyl esterases. Applied Microbiology and Biotechnology 60:489-494.
53. Moukouli M, Topakas E, Christakopoulos P. 2008. Cloning, characterization and functional expression of an alkalitolerant type C feruloyl esterase from Fusarium oxysporum. Applied Microbiology and Biotechnology 79:245-254.
54. Faulds CB, Williamson G. 1995. Release of ferulic acid from wheat bran by a ferulic acid esterase (FAE-III) from Aspergillus niger. Applied Microbiology and Biotechnology 43:1082-1087.
55. Benoit I, Asther M, Sulzenbacher G, Record E, Marmuse L, Parsiegla G, Gimbert I, Asther M, Bignon C. 2006. Respective importance of protein folding and glycosylation in the thermal stability of recombinant feruloyl esterase A. FEBS letters 580:5815-5821.
56. Brenner S. 1988. The molecular evolution of genes and proteins: a tale of two serines. Nature 334:528-530.
57. Dodson G, Wlodawer A. 1998. Catalytic triads and their relatives. Trends in biochemical sciences 23:347-352.
58. Shin HD, Chen RR. 2007. A type B feruloyl esterase from Aspergillus nidulans with broad pH applicability. Applied Microbiology and Biotechnology 73:1323-1330.
59. Mubarak A, Hodgson JM, Considine MJ, Croft KD, Matthews VB. 2013. Supplementation of a high-fat diet with chlorogenic acid is associated with insulin resistance and hepatic lipid accumulation in mice. Journal of agricultural and food chemistry 61:4371-4378.
60. GenBank accession no. **XP_756329.1**

## Claims

1. An enzyme with chlorogenic acid esterase activity and/or feruloyl esterase activity comprising
a. a sequence having at least 85% amino acid identity to SEQ ID NO: 1, and not comprising SEQ ID NO: 3, or
b. a sequence having at least 85% amino acid identity to SEQ ID NO: 1 and not comprising SEQ ID NO: 19.

2. The enzyme of claim 1, wherein the enzyme has chlorogenic acid esterase activity, wherein, preferably, the enzyme has chlorogenic acid esterase activity and feruloyl esterase activity.

3. The enzyme of any of the preceding claims, wherein the enzyme has a molecular mass of about 71 kDa.

4. The enzyme of any of the preceding claims comprising a sequence having SEQ ID NO: 1.

5. The enzyme of any of the preceding claims, wherein the enzyme is obtainable from a basidiomycete.

6. The enzyme of any of the preceding claims, wherein the enzyme is obtainable by expression in yeast, preferably, in *Pichia pastoris.*

7. A nucleic acid encoding the enzyme of any of the preceding claims
a. not comprising SEQ ID NO: 4, or
b. not encoding an enzyme comprising SEQ ID NO: 19.

8. The nucleic acid of claim 7, wherein the nucleic acid is an expression vector comprising the nucleic acid of claim 8 functionally linked to a heterologous promoter, wherein the nucleic acid encoding the enzyme is preferably functionally linked to a heterologous signal sequence.

9. A method of preparing an enzyme of any of claims 1-6 comprising transforming a suitable host cell with the nucleic acid of any of claims 7 or 8, and, optionally, isolating the enzyme.

10. A heterologous host cell comprising the nucleic acid of any of claims 7 or 8.

11. Use of an enzyme with chlorogenic esterase activity and/or feruloyl esterase activity comprising a sequence having at least 85% amino acid identity to SEQ ID NO: 1 for hydrolyzing a substrate and/or generating a product selected from the group comprising
a. a chlorogenic acid, i..e, an ester of quinic acid with a phenylpropanoic acid, preferably, caffeic acid; and/or
b. an ester of a saccharide or C1- or C2-alcohol with phenylpropanoic acid optionally comprising exactly one methoxy on the benzoic ring, preferably, caffeic acid.

12. A method of hydrolyzing a substrate selected from the group comprising
a. an ester of quinic acid with a phenylpropanoic acid, preferably, caffeic acid; and/or
b. an ester of a saccharide or C1- or C2-alcohol with phenylpropanoic acid optionally comprising exactly one methoxy on the benzoic ring, preferably, caffeic acid,
the method comprising a step wherein an enzyme with chlorogenic esterase activity and/or feruloyl esterase activity comprising a sequence having at least 85% amino acid identity to SEQ ID NO: 1 is brought into contact with said substrate under suitable conditions.

13. A method of preparing a product selected from the group comprising
a. quinic acid;
b. phenylpropanoic acid optionally comprising exactly one methoxy on the benzoic ring, preferably, caffeic acid;
c. a saccharide;
d. an ester of quinic acid with a phenylpropanoic acid, preferably, caffeic acid;
e. an ester of a saccharide or C1- or C2-alcohol with a phenylpropanoic acid optionally comprising exactly one methoxy on the benzoic ring, preferably, caffeic acid; and/or
f. a natural material such as food comprising a reduced amount of an ester of quinic acid with a phenylpropanoic acid, preferably, caffeic acid;
the method comprising a step wherein an enzyme with chlorogenic esterase activity and/or feruloyl esterase activity comprising a sequence having at least 85% amino acid identity to SEQ ID NO: 1 is brought into contact with the educts of the catalyzed reaction generating the respective product under suitable conditions, wherein the enzyme is preferably contacted with a natural material such as agro-industrial waste material, such as coffee pulp, destarched wheat bran, and sugar beet pectin or paper pulp;
wherein the product is optionally isolated.

14. A method for reducing dietary risks involving in the consumption of chlorogenic acid, comprising contacting a food product with an enzyme with chlorogenic esterase activity and/or feruloyl esterase activity comprising a sequence having at least 85% amino acid identity to SEQ ID NO: 1.

15. A food product comprising the enzyme of any of claims 1-6.


SEQUENCE LISTING

<110> SternEnzym GmbH&Co. KG

<120> An enzyme with chlorogenic acid esterase activity and feruloyl
esterase activity

<130> STR15531EP

<160> 19

<170> BiSSAP 1.3

<210> 1
<211> 564
<212> PRT
<213> Ustilago maydis

<400> 1
Leu Pro Gln Val Ser Tyr Lys Ala Asp Ala Thr Ala Ser Ala Pro Thr
1               5                   10                  15
Val Lys Leu His Gln Gly Thr Val Arg Gly Leu Ala Asp Asp Asn Tyr
            20                  25                  30
Gly Leu Glu Gln Phe Phe Gly Ile Pro Tyr Ala Lys Pro Pro Val Gly
        35                  40                  45
Ser Leu Arg Phe Ala Lys Pro Gln Pro Leu Gly Pro Ala Ser Ser His
    50                  55                  60
Lys Thr Val Ile Asp Ala Thr Arg Phe Gly Asp Ile Cys Met Gln Thr
65                  70                  75                  80
Val Ala Pro Ser Pro Leu Tyr Asn Met Ser Glu Asp Cys Leu Asn Leu
                85                  90                  95
Asn Val Val Arg Pro Lys Gly Thr Thr Ala Lys Asp Lys Leu Pro Val
            100                 105                 110
Leu Val Trp Ile Tyr Gly Gly Ala Phe Arg Gln Gly Ser Thr Pro Ile
        115                 120                 125
Tyr Asn Ala Ser Glu Leu Val Gln Lys Ser Val Glu Ile Gly Lys Pro
    130                 135                 140
Ile Val Phe Val Ala Ile Ser Tyr Arg Val Gly Pro Phe Gly Phe Ile
145                 150                 155                 160
Gly Gly Ser Glu Ile Ala Asp Ser Asp Ser Ala Thr Ser Asn Ala Gly
                165                 170                 175
Leu Tyr Asp Gln Arg Leu Gly Leu Lys Trp Ile Arg His Asn Ile Gly
            180                 185                 190
Lys Phe Gly Gly Asp Lys Asn Arg Val Thr Leu Phe Gly Gln Ser Ala
        195                 200                 205
Gly Ala Met Ser Ile Ala Leu Gln Asn Phe Ala Tyr Asp Gly Asn Asn
    210                 215                 220
His Gly Leu Trp His Ala Ala Ile Met Asn Ser Gly Gly Ile Ala Pro
225                 230                 235                 240
Gly Pro Leu Leu Thr Pro Lys His Pro Thr Val Glu Gln Ser Phe Lys
                245                 250                 255
Arg Leu Ala Asn Gly Val Gly Cys Thr Gly Gly Ser Leu Leu Arg Cys
            260                 265                 270
Leu Arg Lys Ala Asn Ala Ser Glu Val Gln Thr Val Ala Ser Asn Leu
        275                 280                 285
Thr Ala Gln Ala Gly Gly Thr Phe Pro Ile Pro Gly Ala Leu Ala Trp
    290                 295                 300
Leu Pro Leu Val Asp Tyr Glu Leu Ile Thr Asn Tyr Pro Ser Val Asn
305                 310                 315                 320
Leu Pro Gln Gly Lys Leu Ala Asp Ile Pro Val Ile Gln Gly Asn Ala
                325                 330                 335
Leu Asp Glu Gly Thr Ser Phe Ala Gln Lys Gln Leu Asn Ser Ser Ala
            340                 345                 350

Asp Phe Glu Arg Trp Val Arg Ser Ala Ala Val Ile His Asn Thr Ser
        355                 360                 365
Tyr Thr Glu Gln Ala Leu Gln Lys Val Phe Glu Leu Tyr Pro Asp Val
    370                 375                 380
Pro Glu Gln Gly Ser Pro Phe Tyr Asn Ala Glu Thr Ala Thr Ser Ala
385                 390                 395                 400
Ala Thr Thr Ser Asp Leu Asn Ser Arg Gln Tyr Pro Pro Leu Thr Ser
                405                 410                 415
Asn Gln Tyr Lys Arg Ser Ala Ala Phe Phe Gly Asp Phe Thr Phe Gln
            420                 425                 430
Ala Gln Arg Arg Thr Tyr Leu Lys Ala Ala Thr Leu Gly Trp Lys Lys
        435                 440                 445
Asn Lys Ala Lys Val Trp Ser Tyr Glu Phe Gln Gln Asn Asp Lys Phe
    450                 455                 460
Ala Asn Gly Thr Gly Ser Leu Leu Gly Pro Tyr His Gly Ala Asp Val
465                 470                 475                 480
Lys Tyr Tyr Phe Ile Arg Pro Asp Gly Arg Gln Lys Asp Pro Val Leu
                485                 490                 495
Ala Asp Arg Met Pro Arg Ala Tyr Ile Ser Phe Val Tyr His His Asp
            500                 505                 510
Pro Thr Val Leu Gly Gly Phe Glu Trp Pro Pro Tyr Gly Lys Gly Lys
        515                 520                 525
Lys Leu Leu Gln Met Lys Gly Asp Asn Thr Thr Val Ile Glu Asp Ala
    530                 535                 540
Tyr Arg Lys Glu Ala Met Asp Ala Leu Thr Asn Arg Lys Ala Ala Lys
545                 550                 555                 560
Val Phe Gly Phe

<210> 2
<211> 1695
<212> DNA
<213> Ustilago maydis

<400> 2
cttccacaag tctcttacaa agctgacgcc accgcctctg ctcccacagt caaactccac      60

caaggtactg tgcgcggctt ggccgacgac aattatggcc tggaacaatt ctttggcatt     120

ccctatgcca agccacccgt cggctcgctc cgatttgcca agccacagcc tctcggccca     180

gcctcgtccc acaaaacggt catcgacgct actcgattcg gcgacatctg tatgcagact     240

gttgcaccct cgcctctcta taacatgagc gaagactgtc tcaatctcaa tgtcgttcgt     300

cccaaaggaa caacggccaa ggacaagctt ccggtgctgg tttggatcta tggtggggcg     360

ttcaggcaag gctcaacgcc catttacaac gccagcgaat tggtgcagaa gagcgtcgag     420

attggcaagc cgattgtatt tgttgcgatc agctatcgag tgggtccatt cggcttcatc     480

ggtggatccg agattgctga cagtgactcg gctaccagca acgcagggct gtatgatcag     540

agattgggac tcaagtggat tcgccacaac attggcaagt ttggaggtga caagaacagg     600

gtcactctgt tcggtcagag tgcgggcgcg atgagcattg ctctgcagaa ctttgcctat     660

gatggtaaca accacgggct ttggcatgcc gcgatcatga actcgggcgg aattgctccg     720

ggtccactgt taacgcccaa acaccctacg gtggaacaga gcttcaagag gctggccaat     780

ggcgttggat gtacaggagg atcactgctc cggtgtctgc gtaaagcaaa cgcgagtgag     840

gttcagaccg tggccagtaa tctgactgct caagccggcg gaacatttcc cattcctgga 900

gcgcttgcgt ggttgccact ggtcgactac gagctcatca ccaactatcc cagtgtcaac 960

cttccgcaag gtaaactggc cgacatcccg gtgatccaag gaaacgctct ggacgaagga 1020

acatcttttg cccagaaaca gctcaacagc agcgccgatt tcgaacggtg ggtcagaagc 1080

gctgctgtca tccacaacac ttcgtacacc gagcaagcgc ttcaaaaggt ctttgaactc 1140

tacccagatg ttcctgaaca gggctcgcca ttctacaatg cggagacggc cacttctgct 1200

gctaccacgt cggacctcaa ttcgcgccag tatccacctt tgaccagcaa tcagtacaag 1260

cgttcagcgg cgttctttgg cgacttcact ttccaggcgc agcgacgaac ttatctcaag 1320

gcggcaacct tgggctggaa gaagaacaag gccaaagtat ggtcctacga gttccagcag 1380

aacgacaagt ttgctaatgg tactggatcg ctgttgggcc cataccatgg tgctgatgtc 1440

aagtactatt ttattcgacc tgatggaagg cagaaggacc ccgtcttggc cgatagaatg 1500

cctagggcgt atatcagttt cgtgtatcac catgatccca cggtgctcgg tggtttcgaa 1560

tggccgccct acggaaaagg caagaagctg ctgcagatga aaggtgacaa cacgacagtg 1620

atcgaggatg cctataggaa ggaggcgatg gatgcgctga ccaacaggaa agctgccaag 1680

gtgtttggct tctaa 1695

<210> 3
<211> 21
<212> PRT
<213> Ustilago maydis

<220>
<223> Signal sequence

<400> 3
Met Arg Leu Pro Asn Leu Thr Leu Leu Val Trp Ala Ala Thr Ser Val
1               5                   10                  15
Gly Leu Val Ser Ala
            20

<210> 4
<211> 63
<212> DNA
<213> Ustilago maydis

<400> 4
atgaggctgc ctaatctgac actgctcgta tgggcagcca catccgtcgg cttggtgtcg 60

gct 63

<210> 5
<211> 21
<212> DNA
<213> Artificial sequence

<220>
<223> Primer

<400> 5
tccgttctta gaagccaaac a                                                21

<210> 6
<211> 20
<212> DNA
<213> Artificial sequence

<220>
<223> Primer

<400> 6
gaaagccaag caacaaggtt                                                  20

<210> 7
<211> 38
<212> DNA
<213> Artificial sequence

<220>
<223> Primer

<400> 7
tttttctcga gaaaagagag gctaggctgc ctaatctg                              38

<210> 8
<211> 43
<212> DNA
<213> Artificial sequence

<220>
<223> Primer

<400> 8
tttttctcga gaaaagagag gctgaagctc ttccacaagt ctc                        43

<210> 9
<211> 46
<212> DNA
<213> Artificial sequence

<220>
<223> Primer

<400> 9
tttttgcggc cgcttaatga tgatgatgat gatggaagcc aaacac                     46

<210> 10
<211> 11
<212> PRT
<213> Ustilago maydis

<220>
<223> Tryptic peptide obtained by ESI-MS/MS

<400> 10
Ala Asp Ala Thr Ala Ser Ala Pro Thr Val Lys

1                   5                   10

<210> 11
<211> 14
<212> PRT
<213> Ustilago maydis

<220>
<223> Tryptic peptide obtained by ESI-MS/MS

<400> 11
Phe Ala Lys Pro Gln Pro Leu Gly Pro Ala Ser Ser His Lys
1                   5                   10

<210> 12
<211> 10
<212> PRT
<213> Ustilago maydis

<220>
<223> Tryptic peptide obtained by ESI-MS/MS

<400> 12
His Pro Thr Val Glu Gln Ser Phe Lys Arg
1                   5                   10

<210> 13
<211> 14
<212> PRT
<213> Ustilago maydis

<220>
<223> Tryptic peptide obtained by ESI-MS/MS

<400> 13
Leu Ala Asn Gly Val Gly Cys Thr Gly Gly Ser Leu Leu Arg
1                   5                   10

<210> 14
<211> 11
<212> PRT
<213> Ustilago maydis

<220>
<223> Tryptic peptide obtained by ESI-MS/MS

<400> 14
Val Trp Ser Tyr Glu Phe Gln Gln Asn Asp Lys
1                   5                   10

<210> 15
<211> 9
<212> PRT
<213> Ustilago maydis

<220>
<223> Tryptic peptide obtained by ESI-MS/MS

<400> 15
Glu Ala Met Asp Ala Leu Thr Asn Arg
1                   5

<210> 16

<211> 9
<212> PRT
<213> Ustilago maydis

<220>
<223> Tryptic peptide obtained by ESI-MS/MS

<400> 16
Ala Lys Pro Pro Val Gly Ser Leu Arg
1               5

<210> 17
<211> 585
<212> PRT
<213> Ustilago maydis

<220>
<223> Full-length incl. native signal sequence and P492L

<400> 17
Met Arg Leu Pro Asn Leu Thr Leu Leu Val Trp Ala Ala Thr Ser Val
1               5                   10                  15
Gly Leu Val Ser Ala Leu Pro Gln Val Ser Tyr Lys Ala Asp Ala Thr
            20                  25                  30
Ala Ser Ala Pro Thr Val Lys Leu His Gln Gly Thr Val Arg Gly Leu
        35                  40                  45
Ala Asp Asp Asn Tyr Gly Leu Glu Gln Phe Phe Gly Ile Pro Tyr Ala
    50                  55                  60
Lys Pro Pro Val Gly Ser Leu Arg Phe Ala Lys Pro Gln Pro Leu Gly
65                  70                  75                  80
Pro Ala Ser Ser His Lys Thr Val Ile Asp Ala Thr Arg Phe Gly Asp
                85                  90                  95
Ile Cys Met Gln Thr Val Ala Pro Ser Pro Leu Tyr Asn Met Ser Glu
            100                 105                 110
Asp Cys Leu Asn Leu Asn Val Val Arg Pro Lys Gly Thr Thr Ala Lys
        115                 120                 125
Asp Lys Leu Pro Val Leu Val Trp Ile Tyr Gly Gly Ala Phe Arg Gln
    130                 135                 140
Gly Ser Thr Pro Ile Tyr Asn Ala Ser Glu Leu Val Gln Lys Ser Val
145                 150                 155                 160
Glu Ile Gly Lys Pro Ile Val Phe Val Ala Ile Ser Tyr Arg Val Gly
                165                 170                 175
Pro Phe Gly Phe Ile Gly Gly Ser Glu Ile Ala Asp Ser Asp Ser Ala
            180                 185                 190
Thr Ser Asn Ala Gly Leu Tyr Asp Gln Arg Leu Gly Leu Lys Trp Ile
        195                 200                 205
Arg His Asn Ile Gly Lys Phe Gly Gly Asp Lys Asn Arg Val Thr Leu
    210                 215                 220
Phe Gly Gln Ser Ala Gly Ala Met Ser Ile Ala Leu Gln Asn Phe Ala
225                 230                 235                 240
Tyr Asp Gly Asn Asn His Gly Leu Trp His Ala Ala Ile Met Asn Ser
                245                 250                 255
Gly Gly Ile Ala Pro Gly Pro Leu Leu Thr Pro Lys His Pro Thr Val
            260                 265                 270
Glu Gln Ser Phe Lys Arg Leu Ala Asn Gly Val Gly Cys Thr Gly Gly
        275                 280                 285
Ser Leu Leu Arg Cys Leu Arg Lys Ala Asn Ala Ser Glu Val Gln Thr
    290                 295                 300
Val Ala Ser Asn Leu Thr Ala Gln Ala Gly Gly Thr Phe Pro Ile Pro
305                 310                 315                 320
Gly Ala Leu Ala Trp Leu Pro Leu Val Asp Tyr Glu Leu Ile Thr Asn
                325                 330                 335
Tyr Pro Ser Val Asn Leu Pro Gln Gly Lys Leu Ala Asp Ile Pro Val

340                 345                 350
Ile Gln Gly Asn Ala Leu Asp Glu Gly Thr Ser Phe Ala Gln Lys Gln
        355                 360                 365
Leu Asn Ser Ser Ala Asp Phe Glu Arg Trp Val Arg Ser Ala Ala Val
    370                 375                 380
Ile His Asn Thr Ser Tyr Thr Glu Gln Ala Leu Gln Lys Val Phe Glu
385                 390                 395                 400
Leu Tyr Pro Asp Val Pro Glu Gln Gly Ser Pro Phe Tyr Asn Ala Glu
                405                 410                 415
Thr Ala Thr Ser Ala Ala Thr Thr Ser Asp Leu Asn Ser Arg Gln Tyr
            420                 425                 430
Pro Pro Leu Thr Ser Asn Gln Tyr Lys Arg Ser Ala Ala Phe Phe Gly
        435                 440                 445
Asp Phe Thr Phe Gln Ala Gln Arg Arg Thr Tyr Leu Lys Ala Ala Thr
    450                 455                 460
Leu Gly Trp Lys Lys Asn Lys Ala Lys Val Trp Ser Tyr Glu Phe Gln
465                 470                 475                 480
Gln Asn Asp Lys Phe Ala Asn Gly Thr Gly Ser Leu Leu Gly Pro Tyr
                485                 490                 495
His Gly Ala Asp Val Lys Tyr Tyr Phe Ile Arg Pro Asp Gly Arg Gln
            500                 505                 510
Lys Asp Pro Val Leu Ala Asp Arg Met Pro Arg Ala Tyr Ile Ser Phe
        515                 520                 525
Val Tyr His His Asp Pro Thr Val Leu Gly Gly Phe Glu Trp Pro Pro
    530                 535                 540
Tyr Gly Lys Gly Lys Lys Leu Leu Gln Met Lys Gly Asp Asn Thr Thr
545                 550                 555                 560
Val Ile Glu Asp Ala Tyr Arg Lys Glu Ala Met Asp Ala Leu Thr Asn
                565                 570                 575
Arg Lys Ala Ala Lys Val Phe Gly Phe
            580                 585

<210> 18
<211> 1758
<212> DNA
<213> Ustilago maydis

<220>
<223> Encoding full-length incl. native signal sequence and P492L

<400> 18
atgaggctgc ctaatctgac actgctcgta tgggcagcca catccgtcgg cttggtgtcg      60

gctcttccac aagtctctta caaagctgac gccaccgcct ctgctcccac agtcaaactc     120

caccaaggta ctgtgcgcgg cttggccgac gacaattatg gcctggaaca attctttggc     180

attccctatg ccaagccacc cgtcggctcg ctccgatttg ccaagccaca gcctctcggc     240

ccagcctcgt cccacaaaac ggtcatcgac gctactcgat tcggcgacat ctgtatgcag     300

actgttgcac cctcgcctct ctataacatg agcgaagact gtctcaatct caatgtcgtt     360

cgtcccaaag gaacaacggc caaggacaag cttccggtgc tggtttggat ctatggtggg     420

gcgttcaggc aaggctcaac gcccatttac aacgccagcg aattggtgca gaagagcgtc     480

gagattggca agccgattgt atttgttgcg atcagctatc gagtgggtcc attcggcttc     540

atcggtggat ccgagattgc tgacagtgac tcggctacca gcaacgcagg gctgtatgat     600

cagagattgg gactcaagtg gattcgccac aacattggca agtttggagg tgacaagaac     660

agggtcactc tgttcggtca gagtgcgggc gcgatgagca ttgctctgca gaactttgcc 720

tatgatggta acaaccacgg gctttggcat gccgcgatca tgaactcggg cggaattgct 780

ccgggtccac tgttaacgcc caaacaccct acggtggaac agagcttcaa gaggctggcc 840

aatggcgttg gatgtacagg aggatcactg ctccggtgtc tgcgtaaagc aaacgcgagt 900

gaggttcaga ccgtggccag taatctgact gctcaagccg gcggaacatt tcccattcct 960

ggagcgcttg cgtggttgcc actggtcgac tacgagctca tcaccaacta tcccagtgtc 1020

aaccttccgc aaggtaaact ggccgacatc ccggtgatcc aaggaaacgc tctggacgaa 1080

ggaacatctt ttgcccagaa acagctcaac agcagcgccg atttcgaacg gtgggtcaga 1140

agcgctgctg tcatccacaa cacttcgtac accgagcaag cgcttcaaaa ggtctttgaa 1200

ctctacccag atgttcctga acagggctcg ccattctaca atgcggagac ggccacttct 1260

gctgctacca cgtcggacct caattcgcgc cagtatccac ctttgaccag caatcagtac 1320

aagcgttcag cggcgttctt tggcgacttc actttccagg cgcagcgacg aacttatctc 1380

aaggcggcaa ccttgggctg gaagaagaac aaggccaaag tatggtccta cgagttccag 1440

cagaacgaca agtttgctaa tggtactgga tcgctgttgg gcccatacca tggtgctgat 1500

gtcaagtact attttattcg acctgatgga aggcagaagg accccgtctt ggccgataga 1560

atgcctaggg cgtatatcag tttcgtgtat caccatgatc ccacggtgct cggtggtttc 1620

gaatggccgc cctacggaaa aggcaagaag ctgctgcaga tgaaaggtga caacacgaca 1680

gtgatcgagg atgcctatag gaaggaggcg atggatgcgc tgaccaacag gaaagctgcc 1740

aaggtgtttg gcttctaa 1758

<210> 19
<211> 585
<212> PRT
<213> Ustilago maydis

<220>
<223> NCBI Reference Sequence: XM_751236.1

<400> 19
Met Arg Leu Pro Asn Leu Thr Leu Leu Val Trp Ala Ala Thr Ser Val
1               5                   10                  15
Gly Leu Val Ser Ala Leu Pro Gln Val Ser Tyr Lys Ala Asp Ala Thr
            20                  25                  30
Ala Ser Ala Pro Thr Val Lys Leu His Gln Gly Thr Val Arg Gly Leu
        35                  40                  45
Ala Asp Asp Asn Tyr Gly Leu Glu Gln Phe Phe Gly Ile Pro Tyr Ala
    50                  55                  60
Lys Pro Pro Val Gly Ser Leu Arg Phe Ala Lys Pro Gln Pro Leu Gly
65                  70                  75                  80
Pro Ala Ser Ser His Lys Thr Val Ile Asp Ala Thr Arg Phe Gly Asp
                85                  90                  95
Ile Cys Met Gln Thr Val Ala Pro Ser Pro Leu Tyr Asn Met Ser Glu
            100                 105                 110
Asp Cys Leu Asn Leu Asn Val Val Arg Pro Lys Gly Thr Thr Ala Lys

        115                 120                 125
Asp Lys Leu Pro Val Leu Val Trp Ile Tyr Gly Gly Ala Phe Arg Gln
    130                 135                 140
Gly Ser Thr Pro Ile Tyr Asn Ala Ser Glu Leu Val Gln Lys Ser Val
145                 150                 155                 160
Glu Ile Gly Lys Pro Ile Val Phe Val Ala Ile Ser Tyr Arg Val Gly
                165                 170                 175
Pro Phe Gly Phe Ile Gly Gly Ser Glu Ile Ala Asp Ser Asp Ser Ala
            180                 185                 190
Thr Ser Asn Ala Gly Leu Tyr Asp Gln Arg Leu Gly Leu Lys Trp Ile
        195                 200                 205
Arg His Asn Ile Gly Lys Phe Gly Gly Asp Lys Asn Arg Val Thr Leu
    210                 215                 220
Phe Gly Gln Ser Ala Gly Ala Met Ser Ile Ala Leu Gln Asn Phe Ala
225                 230                 235                 240
Tyr Asp Gly Asn Asn His Gly Leu Trp His Ala Ala Ile Met Asn Ser
                245                 250                 255
Gly Gly Ile Ala Pro Gly Pro Leu Leu Thr Pro Lys His Pro Thr Val
            260                 265                 270
Glu Gln Ser Phe Lys Arg Leu Ala Asn Gly Val Gly Cys Thr Gly Gly
        275                 280                 285
Ser Leu Leu Arg Cys Leu Arg Lys Ala Asn Ala Ser Glu Val Gln Thr
    290                 295                 300
Val Ala Ser Asn Leu Thr Ala Gln Ala Gly Gly Thr Phe Pro Ile Pro
305                 310                 315                 320
Gly Ala Leu Ala Trp Leu Pro Leu Val Asp Tyr Glu Leu Ile Thr Asn
                325                 330                 335
Tyr Pro Ser Val Asn Leu Pro Gln Gly Lys Leu Ala Asp Ile Pro Val
            340                 345                 350
Ile Gln Gly Asn Ala Leu Asp Glu Gly Thr Ser Phe Ala Gln Lys Gln
        355                 360                 365
Leu Asn Ser Ser Ala Asp Phe Glu Arg Trp Val Arg Ser Ala Ala Val
    370                 375                 380
Ile His Asn Thr Ser Tyr Thr Glu Gln Ala Leu Gln Lys Val Phe Glu
385                 390                 395                 400
Leu Tyr Pro Asp Val Pro Glu Gln Gly Ser Pro Phe Tyr Asn Ala Glu
                405                 410                 415
Thr Ala Thr Ser Ala Ala Thr Thr Ser Asp Leu Asn Ser Arg Gln Tyr
            420                 425                 430
Pro Pro Leu Thr Ser Asn Gln Tyr Lys Arg Ser Ala Ala Phe Phe Gly
        435                 440                 445
Asp Phe Thr Phe Gln Ala Gln Arg Arg Thr Tyr Leu Lys Ala Ala Thr
    450                 455                 460
Leu Gly Trp Lys Lys Asn Lys Ala Lys Val Trp Ser Tyr Glu Phe Gln
465                 470                 475                 480
Gln Asn Asp Lys Phe Ala Asn Gly Thr Gly Ser Pro Leu Gly Pro Tyr
                485                 490                 495
His Gly Ala Asp Val Lys Tyr Tyr Phe Ile Arg Pro Asp Gly Arg Gln
            500                 505                 510
Lys Asp Pro Val Leu Ala Asp Arg Met Pro Arg Ala Tyr Ile Ser Phe
        515                 520                 525
Val Tyr His His Asp Pro Thr Val Leu Gly Gly Phe Glu Trp Pro Pro
    530                 535                 540
Tyr Gly Lys Gly Lys Lys Leu Leu Gln Met Lys Gly Asp Asn Thr Thr
545                 550                 555                 560
Val Ile Glu Asp Ala Tyr Arg Lys Glu Ala Met Asp Ala Leu Thr Asn
                565                 570                 575
Arg Lys Ala Ala Lys Val Phe Gly Phe
            580                 585